# EUROPEAN PATENT SPECIFICATION

(11) **EP 2 655 391 B1**
(45) Date of publication and mention of the grant of the patent: **04.09.2019**
(21) Application number: 11810890.1
(22) Date of filing: 16.12.2011
(51) Int. Cl.: C07H 17/08

(54) **NEW AMPHOTERICIN ANALOGOUS COMPOUNDS AND PHARMACEUTICAL COMPOSITIONS CONTAINING THEM**
NEUE AMPHOTERICINANALOGA-VERBINDUNGEN UND DIESE ENTHALTENDE PHARMAZEUTISCHE ZUSAMMENSETZUNGEN
NOUVEAUX COMPOSÉS ANALOGUES D'AMPHOTÉRICINE ET COMPOSITIONS PHARMACEUTIQUES LES CONTENANT

(30) Priority: 21.12.2010 MX 2010014422
(43) Date of publication of application: 30.10.2013
(73) Proprietor: Centro De Investigación Y De Estudios Avanzados Del Instituto Politécnico Nacional (Cinvestav), 07360 Mexico D.F. (MX); Universidad Autónoma del Estado de Morelos, Morelos 62209 (MX); Universidad Nacional Autónoma de México, México D.F 04510 (MX)
(72) Inventor: ANTILLÓN DÍAZ, Armando, Morelos (MX); CARRILLO TRIPP, Mauricio, Guanajuato 36600 (MX); FERNÁNDEZ ZERTUCHE, Mario, Cuernavaca Morelos 62209 (MX); FLORES ROMERO, José David, Cuernavaca Morelos 62209 (MX); JIMÉNEZ MONTEJO, Fabiola Eloisa, Cuernavaca Morelos 62209 (MX); LEÓN BUITIMEA, Ángel, Cuernavaca Morelos 62209 (MX); MORALES NAVA, Rosmarbel, Morelos (MX); OCAMPO MARTÍNEZ, Lilia, Cuernavaca Morelos 62209 (MX); ORTEGA BLAKE, Iván, Morelos (MX); REYES ESPARZA, Jorge Alberto, Cuernavaca Morelos 62209 (MX); RODRÍGUEZ FRAGOSO, María de Lourdes, Cuernavaca Morelos 62209 (MX); SANTIAGO ANGELINO, Tania Minerva, Cuernavaca Morelos 62209 (MX); VARGAS GONZÁLEZ, María Cristina, Mérida Yucatán 97300 (MX)
(74) Representative: Di Giovine, Paolo
(86) International application number: PCT/IB2011/055721
(87) International publication number: WO 2012/085784

(56) References cited:
- EP-A1- 0 434 943
- EP-A1- 0 489 308
- WO-A1-01/91758
- WO-A1-91/09047
- GB-A- 2 055 812
- US-A- 4 041 232
- US-A- 4 783 527
- BRUZZESE T ET AL: "Amide derivatives of partricin A with potent antifungal activity", EUROPEAN JOURNAL OF MEDICINAL CHEMISTRY, EDITIONS SCIENTIFIQUE ELSEVIER, PARIS, FR, vol. 31, no. 12, 1 January 1996 (1996-01-01), pages 965-972, XP004071791, ISSN: 0223-5234, DOI: 10.1016/S0223-5234(97)86175-2

## Description

### Field of the invention.

This invention is useful in the field of medicine and particularly refers to novel analogous compounds of Amphotericin B and to pharmaceutical compositions containing them, which are useful as antibiotics.

### Background of the invention.

Amphotericin B is an antibiotic and antifungal whose molecule is produced naturally by *Streptomyces nodosus*, an actinomycete that was isolated from the soil of the Orinoco River banks in Venezuela in 1955 (Gold et al., Am. Chem Soc., 1971, 93, 4560-4564). Two chemical forms exist: the Amphotericin A, without clinical application and with macrolide chemical configuration, and Amphotericin B (AmB). Its name is originated from the amphoteric properties of the chemical agent.

AmB is a member of a family of nearly 200 polyene macrolide antibiotics, whose structure was unambiguously determined in 1970 through X-ray crystallography studies (Ganis et al., J. Am. Chem. Soc., 1971, 93, 4560-4564).

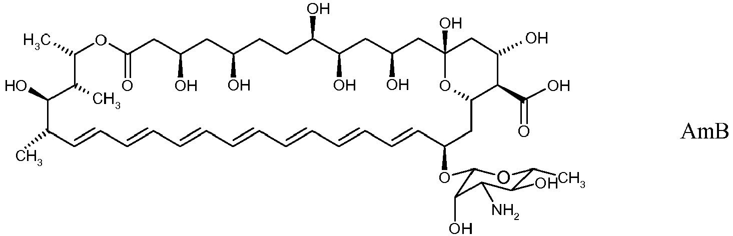

AmB is a cyclic amphiphile composed of two long chains, a poly hydroxy which is hydrophilic and the other a hydrophobic polyene of seven double bond links conjugated with E geometry. These two chains are attached to both ends causing a macrolactone. One end is called "polar head," which contains a carboxyl function plus an amino carbohydrate unit (3-amine-6,6-dideoxymanose) also known as mycosamine ring, linked to the main ring by a glycosidic bond. The other end known as "tail" is characterized by three methyl groups and a hydroxyl group.

The amino and carboxyl groups of the polar head are protonated and deprotonated respectively at a physiological pH (7.4). Under these conditions, AmB is poorly soluble in water at concentrations below 1 mM; at higher concentrations it forms oligomers that precipitate in solution. However, extreme pH values, under 2 and over 11, its solubility in this liquid augments greatly, hence, its name of amphotericin, as already mentioned, adverts to both solubility behaviors (Vandeputte et al., J. Infect. Dis., 1986, 154, 76-83). In 1988, Nicolaou reported the total synthesis of this antibiotic (Nicolaou, et al., J. Am. Chem. Soc., 1988, 110, 4696-4705).

Microbiological tests developed for the natural AmB showed a broad antifungal spectrum that included species of *Aspergillus* and *Candida.* These results also showed the ability of AmB to control infections caused by *Candida albicans* in chicken eggs and mice, and its antifungal effect at lower concentrations compared to Nystatin (Nys) and Rimocidin, two antifungal antimycotics whose activity is similar to that of AmB.

Subsequent studies have shown that AmB has a broad fungicide spectrum and fungistatic action against various yeasts, dimorphic fungi, dermatophytes and opportunistic fungi (D'Arcy, P.F. & Scout E. M., Antifungal Agents, Prog Drug Res, 1978, 22, 93-147); it even shows a selective activity against some protozoa and viruses (Kessler, et al., Antimicrob Agents Chemother. 1981; 20(6):826-33).

Moreover, it was observed that in recent years, systemic fungal infections have increased dramatically, mainly resulting from opportunistic fungi. They are called opportunistic because they normally live in the oral, nasal, gastrointestinal and vaginal mucous of humans (Quindós, G., Revista Iberoamericana de Micología, 2002, 19: 1-4).

The frequency of opportunistic invasive fungal infections has increased significantly over the past two decades (Pfaller et al., Crit Rev Microbiol. 2010; 36(1):1-53). The increase in infections is associated with excess morbidity and mortality, and is directly related to the increase in patients who are at risk for serious fungal infections, even those patients who undergo a blood transfusion and marrow transplantation, solid organ transplant and major surgery (especially, gastrointestinal surgery), patients with AIDS, neoplastic disease, advanced age, as well as patients receiving immunosuppressive therapy, and premature infants (Hof, Eur J Clin Microbiol Infect Dis. 2010;29(1):5-13; Marr, Curr Opin Oncol. 2010;22(2):138-142; Khambaty et al., Emerg Med Clin North Am. 2010;28(2):355-36).

Contemplating the complexity of the patient population at risk of infection and the diversity and increase of fungal pathogens, opportunistic mycosis poses a challenge in their diagnosis and therapy. The known causes of opportunistic mycosis include *Candida albicans, Cryptococcus neoformans,* and *Aspergillus fumigatus* (Almirante, et al., J. Clin. Microbiol. 2005, 43: 1829-1835). The estimated annual frequency of invasive fungal infections resulting from these pathogens is 72-228 infections per million individuals to *Candida* species, 30-66 infections per million individuals to C. *neoformans*, and 12-34 infections per million individuals to *Aspergillus* species (Anuarios de Morbilidad. Dirección General de Epidemiologia, Secretaria de Salud. http//www.dgepi.salud.gob.mx/anuario/index.html). The new and "emerging" fungal pathogens include *Candida* and *Aspergillus* species aside from C. *albicans* and *A. fumigatus*, a fungus-like opportunistic yeast (eg. *Thrichosporon* and *Rhodotorula* species), the *Zygomycetes*, hyaline molds (eg. *Fusarium* and *Scedosporium*), and a wide variety of dematiaceous fungi (Pfaller, et al., J. Clin. Microbiol. 2004, 42: 4419-4431; Pfaller, et al., Clin. Infect. Dis. 2006, 43: S3-S14). Infections caused by these organisms range from catheter-related fungemia and peritonitis, more localized infections (eg, those involving the lungs, skin and sinuses), and widespread hematogenous dissemination.

The diagnostic and therapeutic advances emerged in recent decades have led to an augment in the number of transplant patients, the survival of onco-hematological patients, those suffering from chronic diseases, premature infants, immunocompromised, burned, critically ill patients, trauma patients, surgical patients undergoing major surgery, etc. All which encourage the emergence and growth of a population at high risk for fungal infections.

In areas of critical care medicine nosocomial, infections by *Candida spp* range from 25% to 50%. This circumstance results because in these areas the most susceptible patient population is concentrated (Blot, et al., Am J Med 2003, 113: 480-485; Tortorano, et al., Eur J Clin Microbiol Infect Dis 2004, 23: 317-322). After an excessive and sustained inflammatory response critically ill patients admitted to intensive care areas suffer an immunosuppression secondary to cellular immunity dysfunction, impaired monocytes and neutrophils response, which make of them a population especially vulnerable to opportunistic infections such as candidemy from the 14-day stay.

Over time there have been gradual changes in the species of *Candida* that produces the infection, and although the C. *albicans* is the most prevalent, *C. parapsilosis, C. glabarata, C. tropicalis* and C. *krusei* have gained greater prominence, depending on the country studied, the type of patient, etc.

The term candidiasis is used for numerous infections resulting from yeasts of *Candida* genus. The C. *albicans* is among them the most important etiologic agent in this type of pathology. In the microscope it is seen as round cells, oval (3-7 mm in diameter), or gemmates that are linked together to form pseudomycelia or that elongate to form mycelium (Bonifaz, A., Micología Medica Básica, 2nda. Ed., México, D. F., 2002, 498-500). The species of *Candida albicans* genus produce germ tubes. In Sabourand agar they grow into white, soft, creamy and smooth colonies.

The three fungi pathogenic effects for its medical importance include mycotoxicosis, hypersensitivity diseases and colonization of tissues (Murria, et al. Medical Microbiology 2002, 4a. Ed. St. Louis; Mosby); the latter is the primary means by which yeasts of the *Candida* genus produce their pathogenic action in man and animals. The adherence of C. *albicans* is the first step in colonization and monocutaneous tissue invasion, which is probably mediated by the interaction of the surface glycoproteins of the yeast with the host epithelial cell. The germ tubes - mycelium or pseudomycelia (depending on the species)- is produced then, which penetrate directly into the epithelial cell. The adherence continues with the production of hydrophilic and proteinase enzymes, phosphatases and phospholipases. The fungi proliferate after entering the epithelial cell. Generally, the non-adherent *Candida* species is non-pathogenic (Bennet, et al., Clinical Microbiology Review 2003, 16, 497-516).

The presence of *Candida albicans* in certain infectious processes is given by certain predisposing factors. In this sense the main factors are:
1. Damage to the skin integrity by maceration of tissues, wounds, abrasion by thermal or chemical burns and the presence of vascular catheters.
2. Mucocutaneous barrier disruption by diabetes, the use of antimicrobial agents, the incidence of smoke irritation, the use of cytotoxic drugs, corticosteroids, vagotomy resulting in the increase of gastric pH, nasogastric intubation, or diaphragms.
3. Nutritional or hormonal imbalance caused by diabetes, oral contraceptives, pregnancy, malnutrition, and uremia.
4. Decreased number of phagocytic cells because of leukemia, granulomatosis, radiation therapy, or chemotherapy to fight cancer.
5. Intrinsic defects in the phagocytic cell performance because of chronic granulomatous disease and myeloperoxidase deficiency, and
6. Impaired phagocytic function caused by uremia, viral diseases and the use of corticosteroids and antimicrobial agents such as aminoglycosides and sulfonamides.

Briefly, the main known clinical forms of candidiasis infections are: (a) genital Candidiasis; (b) oral Candidiasis (thrush, mouget, or toad); (c) esophagitis, which usually is a result of oral Candidiasis; (d) intertrigo; (e) onicomicis by *Candida*; (f) granulomas; (g) chronic mucocutaneous Candidiasis; (h) urinary Candidiasis; (i) deep systemic Candidiasis, such as bronchopulmonary Candidiasis, endocarditis, and meningoencephalitis; and (j) Candida sepsis.

The optimal treatment strategy for candidal infections is controversial. Amphotericin B in sodium deoxycholate has been used as a standard treatment for five decades, but its toxicity and limited efficacy has led to the need for new alternative drugs. Fluconazole is effective and safer as amphotericin B for the treatment of candidemia, albeit the reduced susceptibility of some species such as *C. glabatra* and C. *krusei* may limit its use in places where these species are prevalent. More recent options for patients with candidemia or invasive candidiasis include broad-spectrum azoles such as voriconazole and echinocandins (caspofungin, micafungin, and anidulafungin). Large randomized studies have been performed to compare these new agents with existing treatment regimens. The first one compared caspofungin with amphotericin; the second compared voriconazole with a short course of amphotericin B followed by fluconazole; and more recently, anidulafungin compared with fluconazole, whereas micafungin was evaluated both with liposomal amphotericin B (L-AmB), and with caspofungin. However, these studies conclude that the echinocandins have been established as highly effective and safe drugs, and can be an alternative treatment to conventional amphotericin B or azole antifungals; among them anidulafungin may even be superior to fluconazole with regard to clinical efficacy (Kullberg, et al., Lancet 2005, 366: 1435-1442; Kuse, et al., Lancet 2007, 369: 1519-1527; Tanger et al., Saudi Med J 2008;29(5):728-733; Pappas et al., Clin Infect Dis 2007; 45: 883-893; Reboli, et al., N Engl J Med 2007, 356: 2472-2482).

The mechanism of action of AmB is not fully clarified; however, it is known to interact directly with membrane lipids and modifies their permeability (Venegas, et al., Biophys J 2003, 85: 2323-2332), which causes a loss of cellular homeostasis and death. Furthermore, it presents a selective activity of the membranes containing sterols over those that do not contain them (Lampen, et al. J. Bacterial. 1960, 80. 200-206). This fact is the basis of the toxicity associated with the clinical use of AmB.

The action of AmB in cells from various sources such as fungi, mammals, bacteria, protozoa, its selectivity and its dependence on various physicochemical conditions have led to extensive studies of polyene antibiotics to understand the molecular action mechanisms that lead to an insight of transmembranal phenomena in the biological cell. Also, as earlier mentioned polyene antibiotics are widely used therapeutically. Frequently it is the only antibiotic of choice, and its wider use is limited by considerable toxic side effects.

Currently there are three types of antifungal agents available for treating systemic infections: the polyenes, azoles, and echinocandins. However, the ideal antimycotic for the treatment of these infections continues to elude scientists (Chapman, et al., Trans Am Clin Clim Ass 2008, 119:197-216), and polyenes still take the lead because there is virtually no risk of developing resistance to the antibiotic by the microorganism.

To reduce collateral toxicity of AmB a lipid formulation was patented in 1990 that markedly decreased the toxicity associated (Proffitt, *et al.* USPat 6,770,290); but the use of this formulation has not fully resolved the main problem of collateral toxicity, and it has also increased the cost of the corresponding therapy.

Therefore, the search for derivatives of AmB -the most widely used polyene therapy for systemic infections- has spanned more than five decades.

Regarding the background in the search for derivatives, we can mention the Borowski group with their main seat in the Gdansk University of Technology. This group developed the replacement of lineal amides in the carboxyl function comparing their antifungal and hemolytic activity in yeast and erythrocytes. (Borowski, 1982) (Jarzebski, et al., J Antibiotics 1982, 35:220-229). Various groups have also considered alkyl derivatives by substitution both in sugar and carboxyl function (Ibagrimova, et al., Biochem Biophys Acta 2006, 1758:29-37). The Murata group of the University of Osaka even synthesized a compound by linking the sugar and the carboxyl group seeking a greater rigidity of the molecule (Matsumori, et al., J. Am Chem Soc., 2005, 127, 10667-10675*).* Another entity working with AmB derivatives is the Carreira group at the Institute of Technology of Zurich, who performed a mycosamine bis-alkylation, which apparently procured an improvement in selective toxicity (Carreira, 2006) (Paquet, et al., Org Lett 2006, 8: 1807 -1809). Recently, the Zotchev group of the Gause Institute of New Antibiotics of Russia made many substitutions in both the mycosamine and the carboxyl function and characterized them in their antifungal and hemolytic activity, and sometimes the median lethal dose in mice (Preobrazhenskaya, et al., J Med Chem 2008, 52:189-196).

All these studies seek to increase the selectivity and maintain the power of the parent molecule. Such is the domination of polyene antibiotics in the therapeutic use delineated above, and such is the interest in understanding the mechanism of action of these antibiotics that for over five decades several studies have been performed to elucidate these mechanisms. Here, we can highlight the following:
1. Transmembranal pore formation incorporating sterol as an integral part of the channel. This model propounds that AmB forms a complex with sterol (B. De Kruijff, 1974), in which the sterol molecule fits together in the ring part of the AmB molecule, and this complex could be extended to a circular array of 8 units. The inside of the complex is hydrophobic owed to the hydroxyl groups of AmB molecules, while the exterior is hydrophobic because of the intercalated double bonds of the molecules of AmB and the sterol molecules._This type of complex produces a single pore through the lipid membrane. For a double pore two of these simple pores on both sides of the membrane are necessary. In the bilayers composed of two monolayers the pore is formed by a simple addition of AmB or one of its derivates over one side of the membrane, and this produces univalent cation selective conductances. The addition of AmB or one of its derivates over both sides of the membrane results in the association of these simple pores that produce selective conductances for univalent anions (Finkelstein, et al., Membranes 1973, 2:377-408; y Kleinberg, et al., J. Membr Biol 1984; 80:257-269). This model assumes the presence of sterol in the membrane as a prerequisite for the action of polyene antibiotics.
   Each molecule of AmB can be assumed as a plane that is inserted into the membrane having a hydrophilic and a hydrophobic side, and a bump on the membrane. The hydrophobic side corresponds to the amphipathic chain, the protuberance of the amino sugar and the hepatene hydrophobic face. Finkelstein's model assumes that the sterol is intercalated between two monomers bonding the sugar parts. This generates a polar interior of the pore with a non polar exterior. The ring of hydroxyl groups can be united by hydrogen bonds with an identical structure from the other side of the membrane to form a double pore. However, the hydroxyl ring can be in contact with the opposite aqueous phase, and then the simple pore would cover the whole membrane. This is possible because the width of the membrane may vary: the structure of the lipid molecules may change resulting in a greater or reduced width of the membrane. However, the sensitivity of the membranes to the effect of appending AmB or Nys to a single face depends on their length. Particularly, membranes with more than 18 carbons in the fatty acid chains are insensitive to the effect of appending AmB and Nys to one side (Finkelstein, 1984) (Kleinberg, *et al, supra).*
   The work of Bruyan and McPhie in 1996 reported the action of a single side of AmB in membranes with ergosterol and cholesterol. Their results show the formation of well-defined ion channels in both membranes and ergosterol in the membranes with cholesterol. They also report that although the channels have similar conductances the opening times are different, 100 times higher in ergosterol membranes. They also found that it requires approximately the same concentration of AmB to form channels in both membranes. Another interesting observation is the dependence between the applied voltage and the number of open channels, as the numbers of channels augments when applying a positive voltage and diminish when applying a negative voltage. The similarity in the observed properties of membranes with ergosterol or cholesterol leads to the conclusion that the action of one side of the AmB ion channel with the same molecular structure in both types of membranes and the pore structure is that of the classic pore of AmB with a different effectiveness of AmB in ergosterol and cholesterol membranes, because of the difference in time of the channel opening (Brutyan, 1996) (Brutyan, et al., J Gen Physio 1996, 1107:69-78).
   Although the classical model of AmB channel (and several of its derivatives) contemplates sterols as an integral part thereof, cationic selectivity and anionic selectivity channels have been observed (HsuChen, et al, Biochem Biophys Res Commun 1973, 51:972-978) in membranes without sterols. Some authors have adverted to these channels as protochannels, defining them as structures that are responsible for antibiotic activity but that may pharmacologically evolve the active channel when sterols are present in the membrane (Cohen, et al., Biochem Biophys Acta 1992, 1108:49-58). However, the work of Cotero and colleagues (Cotero, et al., Biochim Biophys Acta 1998, 1375:43-51) and Venegas and colleagues (Venegas, *et al. supra*) show that the AmB channels in membranes free of sterol are the same that in the presence of it. The works of Cotero and colleagues (Cotero *et al., supra)* show that the conductivities of the unitary channel are independent of sterol, suggesting that the molecular structures formed in the presence and in the absence of sterol are the same.
2. Dependent membrane insertion of the aggregation of the drug in aqueous solution. An alternative model assumes that while the drug acts through the transmembranal pores, the selectivity of membranes with ergosterol (fungi) with respect to cholesterol (mammals) is that its insertion in the cellular membrane is sensible to the aggregation presented by AmB, or its derivatives in aqueous solution from the bloodstream. The work of Huang and colleagues (Huang, et al., Biophys J 2002, 83:3245-3255) withal shows that despite the type of AmB solution -monomeric or aggregated- it can form channels in ergosterol membranes. However, AmB in a monomeric state cannot form channels in membranes without sterol or with cholesterol. When AmB is in a dimeric state it is able to form channels in membranes with cholesterol, and only when present in highly aggregated states can it form channels in membranes without sterol. These results allow proposing that the aggregation state of AmB plays a key role in the insertion of the drug in the membrane, previous to the formation of the channels.
3. Selectivity of the drug by modulation of the membrane structure. In subsequent work (Venegas, *et al, supra),* a comparison is made between the channels present in the membranes with cholesterol and ergosterol, and between different concentrations of antibiotic required for the expression of the channels. The well-defined conductivity spectrum allowed classifying six types of AmB channels. For each type the conductivities observed were very similar for membranes with or with sterol and with different lipid compositions, so that the supramolecular structure of the channels seems to be the same in all cases. However, since the presence of sterols augments the potency of the antibiotic, it is necessary to compensate the lack of sterols with a higher concentration of AmB, thus having different thresholds for the channels expressions in the cases studied. It also shows how cholesterol increases the presence of AmB in the membrane by promoting the expression of large channels. This would accord with the results of Cohen that the small channels are the protochannels, which later convert into the aqueous channels (large channels). The abundance of AmB in the membrane with ergosterol led to the apparition of large channels without the disappearance of the small channels.
   This type of evidence casts doubts on the role of sterols in the formation of the channels. So that, models have been proposed in which the sterols are not part of a channel, but as it is known they change the structural properties of the membrane, which in turn modifies the presence of polyene in it. Certain works such as Millhaud and col., (Millhaud, et al., Biochim Biophys Acta 2002, 1558:95-108) make a comparison of the AmB uptake in membranes without sterol and ergosterol. In this work, the authors consider that the aggregates of AmB in water consist of a set of plane dimers. The results show that in membranes without sterol the aggregates of AmB remain on the surface of the membrane and are probably slightly attached to it by hydrogen bonds mediated by water. However, in membranes with ergosterol the AmB aggregates are embedded in the membrane, and most of them have a hole in the center. While these are large aggregates that are deposited on membranes supported for a period of days, they may indicate that the drug interaction with the membrane surface is different for each sterol.
   Supporting the idea that the structure of the membrane is possibly responsible for the selectivity of the drug by the sterol, we can advert to the work of Zumbuehl and col. (Zumbuehl, et al., Org Lett 2004, 6:3683-3686), who consider AmB as a potential reporter of the physical state of a membrane. They establish that there is an upper limit on the ordering of the membrane whereto AmB is no longer able to insert itself. Their results show that AmB has a preference for the coexistence of liquid-disordered and liquid-ordered (1d+1o) phases. These observations are consistent with the model of AmB in which the selectivity of the membrane is given by its different structure resulting from the different sterols present. There are works that support the above idea such as that of Lambing and col., (Lambing, et al., Biochim Biophys Acta 1993; 1152:185-188), which studies the effect of temperature on the aggregation state and activity of AmB. It is known that when increasing the temperature the AmB solution changes from a state of more aggregation to a state of less aggregation. In a 1993 work, they observed the change in the flow of K+ by increasing the temperature in membranes with cholesterol and ergosterol. The results suggest that in membranes with cholesterol the flow K+ decreases four times while augmenting 10°C. However, in membranes with ergosterol the permeability effect is not as elucidate. Withal there seems to be a maximum of activity between 25 and 30°C. They conjecture that this more complicated behavior may be because ergosterol membranes probably are sensitive to both the aggregate and monomers and thus, different types of channels are formed with different permeability characteristics. This would accord with the model of AmB in which the membranes with cholesterol are sensitive to the AmB aggregates while the membranes with ergosterol are sensitive to aggregates and monomers.
4. Cell membrane disruption. Bolard and col. (Bolard, et al., Biochem Biophys Acta 1980, 599:280-293) proposed another alternative model, which assumed that the effect of polyene antibiotics is to produce disruptions of the cell membrane that cause its loss of integrity, which leads to the loss of K+ and a consequent lethality. This mechanism has recently been considered a mechanism of action in peptides antibiotics and is called "carpet model," in which the amphipathic molecules reach the membrane either as monomers or oligomers. These attach then to the surface of the membrane with its hydrophobic part, leaving the hydrophilic part on the solution. When a threshold concentration of monomers is reached, the membrane is permeated and transmembranal pores may form. In some cases, this process may lead to the disintegration of the membrane.

Application of the models in the design of derivatives. The various models presented have led to strategies for procuring derivatives of AmB with similar antifungal potency but with a reduced collateral toxicity, thus improving their therapeutic use. Many derivatives have been developed by incorporating substituents on the sugar group which should differentiate with a greater selectivity the association with ergosterol and cholesterol. It has also been tried to synthesize compounds in which polyene is attached to a sterol, and thereby augmenting its potency (Matsumori, et al., Chem Biol 2004, 11:673-679).

Hence, multiple agents with different action mechanisms are available, which opens an opportunity for combination therapy. The advantage of combination therapy includes the potential for synergy, a broader coverage and a possible decrease of drug resistance (Jonson, et al., Antimicrob Agents Chemother 2004, 48:693-715; Patterson, TF, Pediatr Infect Dis J 2003; 22: 553-556; y Sobel, JD, Clin Infect Dis 2004; 39:S224-S227).

No essays of combination therapy have been reported in children. There are data concerning the potential for antagonism to fluconazole and amphotericin B combined, based on *in vitro* studies, but this has not been sustained in *in vivo* studies. A multicentric, randomized and controlled study on fluconazol and placebo versus amphotericin B was reported for neutropenic candidemia in adult patients. The patients on the combination group showed a success rate of average improvement (69% versus 56%, P<0.04) (Rex, et al., Clin Infect Dis 2003; 36:1221-1228). The combination of echinocandin and fluconazole is currently under study. An *in vitro* study of the combination of caspofungine and fluconazole documented a reduced caspofungine activity (Roling, et al., Diagn Microbiol 2002, 43:13-17). An *in vivo* study in mice not showed a better depuration of C. *albicans* from kidney compared to fluconazole alone (Graybill, et al., Antimicrob Agents Chemother 2003; 47:2373-2375).

Generally, the incidence of adverse reactions by treatment with amphotericin B and sodium deoxycholate is high. There are actually two types of adverse reactions; a) immediate reactions: in most patients is very frequent the occurrence of fever, chills and shivering during the infusion of the drug in the first week, sometimes accompanied by headache, vomiting and hypotension. These effects can be reduced by prior administration of antipyretics, antihistamines and / or antiemetics. b) In relation to the dose and / or duration of the treatment: the most important adverse effect and the main factor limiting its use is nephrotoxicity; particularly, when amphotericin B is used in combination with other potentially nephrotoxic agents (aminoglycosides, cyclosporine, etc.), or in situations where renal damage is of paramount concern. The kidney damage is usually reversible upon discontinuation of the drug; albeit it may take several weeks for its normalization (Deray, G., J Antimicrobial Chemo 2002; 29, suppl. S1:34-41; Golmand, et al., J Pediatr Hematol Oncol 2004; 26(7):421-6).

Nephrotoxicity can be reduced by ensuring an adequate hydration of the patient. Over 25% of patients develop hypokalemia and hypomagnesaemia. The development of normocytic normochromic anemia as a result of the inhibition of erythropoietin is rare (Poncio-Méndez, Rev Inst Med Trop S Paulo 2005; 47(Suppl. 14)).

Thrombophlebitis associated with the intravenous administration of amphotericin B with sodium deoxycholate is also frequent. Extravasation of the drug may cause tissue necrosis. Anaphylactic reactions are rare. A rapid drug intravenous administration (within 60 min) may trigger cardiac arrhythmias and cardiac arrest. Intrathecal administration may cause nausea, vomiting, urinary retention, headache, radiculitis, paresis, paresthesia, visual disturbances and chemical meningitis. The main contributions of other formulations of amphotericin B in lipid and liposomal complex is a better tolerance and most important, its lower nephrotoxicity, allowing a higher daily dose and a total dose over a shorter time. Amphotericin B in lipid complex is better tolerated than amphotericin B with deoxycholate with a lower incidence of adverse effects related to the infusion, however premedication is advised (Lemke, et al., Appl Microbiol Biotechnol 2005; 68:151-61).

Amphotericin B and analogue polyene antibiotics as already adverted to alter the structural and functional integrity of a variety of biological systems. The specific effect of amphotericin B has been attributed to the ability of the drug to interact reciprocally with the limit of the sterol membrane. It has been suggested that the polyene-sterol interaction causes a reorganization of the membrane structure, augmenting its permeability. In erythrocytes amphotericin B enhances the membrane permeability to various electrolytes and nonelectrolytes, and an increased entry of K+ and the cell glycolysis resulting from the stimulation of amphotericin B to the cation pump, inducing hemolysis (Lemke, *et al., supra).*

As adverted to briefly in the foregoing, in an attempt to combat the toxic effects of amphotericin, in recent years several researchers have ventured into the synthesis of new analogues from amphotericin B. It has been observed that the chemical modifications made led to the suppression of the charge on the exocyclic carboxyl group reduced toxicity and an improved antifungal specificity (Cheron, et al., Biochem Pharmacol 1998; 37: 827-836). Further improvements were made also by derivatization with added sugars (Szlinder-Richert, et al., Biochem Pharmacol 2001, 1528: 15-24). This based on previous studies of chemical modifications that have shown that a positive charge on the amino sugar is important for the antifungal activity of amphotericin B.

In 2006 Paquet and Carreira generated low-molecular weight analogues with greater activity by structural changes in the mycosamine region of the AmB molecule. These analogues showed to be more active than AmB against *Saccharomyces cerevisiae* and amphotericin resistant *Candida albicans* strains. They also presented a lower haematotoxicity when compared with AmB. Specifically, the derivative mycosamine bis (aminopropane) showed the highest antifungal activity and the lowest haematotoxicity. The results obtained suggest that alkylations made in the mycosamine region of AmB with two aminopropane groups allow the generation of analogues with a significant improvement in biological activity (Paquet, et al. Org Lett 2006, 8(9): 1807-1809).

Similarly, the international patent application PCT/EP07/001468 of Carreira, R., and col. describes polyene macrolide derivatives with antifungal activity; however, these derivatives have low values of selectivity regarding fungal cells when seeking a reduction in collateral cytotoxicity. Also, in this patent application a bialkylation of the amino group is performed, a situation which is not contemplated or performed in the present invention.

Based on the foregoing, in the state of the art there is an urgent need to have analogues of amphotericin B that are effective in their antibiotic properties, particularly as a broad-spectrum antifungals with low frequency of intrinsic or acquired resistance, which are manageable through oral or intravenous formulations for ease of application, and to provide a higher level of selectivity regarding the cells to be fought with a lower toxicity than that of amphotericin B natural or synthetic and the AmB analogous compounds currently on the market. It is also appropriate for this alternative to be of low cost.

In search of satisfiers of the need raised in the preceding paragraph, the inventors found after an intensive research work that changes in the COOH group present in polyene macrolide without concomitant replacement of the amino group of deoxysugar, e.g., mycosamine of amphotericin, or the lactone ring modification, give rise to derivatives that are more selective in their antibiotic action on fungal cells than in those of mammals, both in erythrocytes and kidney cells. Moreover, these new analogous compounds of AmB developed by the inventors show a well-defined behavior in their unit transmembrane channels that ratify their much less effectiveness in membranes containing cholesterol.

### Objectives and summary of the invention.

The present invention is defined in the appended claims. All aspects and embodiments which are labelled as "aspects or embodiments of the invention" but not covered by the claims are merely aspects of the present disclosure and do not form part of the invention. Any disclosure going beyond the scope of said claims is only intended for illustrative purposes.

The objective of the present disclosure is, *inter alia*, to provide polyene macrolide derivatives according to formula (A): 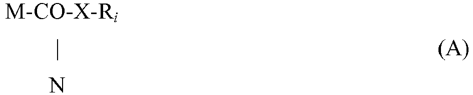 where M is a macrocyclic lactone ring; N is polyene sugar, substituted or unsubstituted; X is independently selected from O, S, N and NH; R is independently selected from an alkyl, cycloalkyl, heterocycloalkyl aryl, heteroaryl, arylalkyl, and heteroarylalkyl group; and *i* is an integer number from 1 to 3, with the condition that it has a negative charge or the character of zwitterions is restored; or a pharmaceutically acceptable salt thereof; useful as antibiotics.

An alternative embodiment of this invention provides analogous compounds of amphotericin B, whose design has focused on the replacement of the carboxyl group exposed to the extracellular medium; this substitution changes the interaction of the drug with the polar heads of the lipids. Contemplating these aspects, the inventors performed the synthesis of a series of analogues of AmB, as these have the advantage of being produced by a specific reaction of the carboxyl function, which is in the polar head of the molecule and the region of interest. This ensures that the rest of the molecule remains without structural changes. The choice of the amine used to synthesize the derivatives was performed according to the criterion that the resulting amide should cause the interactions adverted to in a favorable way to increase the channel stability and thus, optimize the antifungal activity.

An important aspect of the analogues of this invention is that the derivatives obtained from amphotericin B show the same pharmacological properties but with reduced side effects. Hence, it provides a series of analogues that has different efficacy and potency compared to amphotericin B as fungicide against *Candida albicans.* In addition, the analogues provided by the present invention produce fewer toxic effects on human renal cells and less toxicity in human erythrocytes (hemolysis).

In another aspect of this invention pharmaceutical compositions are provided comprising at least one analogue of AmB and a pharmaceutically acceptable carrier.

In still another aspect of the invention pharmaceutical compositions for combination therapy are provided comprising at least one analog of AmB, a coadjuvant agent and a pharmaceutically acceptable carrier, where the coadjuvant agent is selected from antifungal, antipyretic, antihistamine or antiemetic components.

### Brief description of the figures.

**Figure 1****.** Graph showing the effect of analogous compounds of the present invention at various concentrations on cells of *Saccharomyces cerevisae* FY833(SC) with reference to AmB and dimethyl sulfoxide (DMSO).
**Figure 2****.** Graph showing the effect of the compounds of the inventions at various concentrations on human renal cells 293Q. (ATCC CLR-1573) with reference to AmB and DMSO.
**Figure 3****.** Graph showing the relative selectivity of the action of the compounds of the present invention in the viability of *Saccharomyces cerevisae* FY833(SC) cells compared to viability of renal human cells 293Q (ATCC CLR-1573) called EHFK.
**Figure 4****.** Graph showing the antifungal activity of amphotericin B and the analogue A21 of the invention against *Candida albicans* ATCC 10231.
**Figure 5****.** Graph showing the antifungal activity of amphotericin B and the analogue A21 of the invention against *Candida kruzei.*
**Figure 6****.** Graph showing the antifungal activity of amphotericin B and the analogue A21 of the invention against *Candida albicans* ATCC 752.
**Figure 7****.** Graph showing the effect of amphotericin B and the derivative A21 of the invention on human erythrocytes.
**Figure 8****.** Graph showing the effect of AmB and the analogue A21 of the invention on human renal cells 293Q.
**Figure 9****.** Graph showing the channels produced by AmB in the lecithin membrane of chicken egg containing 30 mol % cholesterol at a concentration of 6 µmol at a temperature of 30°C and with an applied potential of 200 mV.
**Figure 10****.** Graph showing the channels produced by AmB in the lecithin membrane of chicken egg containing 30 mol % ergosterol at a concentration of 3 µmol at a temperature of 30°C and with an applied potential of 200 mV.
**Figure 11****.** Graph showing the channels produced by the derivative A21 of the invention in the lecithin membrane of chicken egg containing 30 mol % cholesterol at a concentration of 80 µmol at a temperature of 30°C and with an applied potential of 200 mV.
**Figure 12****.** A graph showing the channels produced by the derivative A21 of the invention in the lecithin membrane of chicken egg containing 30 mol % ergosterol at a concentration of 6 µmol at a temperature of 30°C and with an applied potential of 200 mV.
**Figure 13****.** Graph showing the extinction coefficient of the derivative A21 of the invention as a function of the concentration.
**Figure 14****.** Graph of the points obtained by flow cytometry.

### Detailed description of the invention.

In the development of the analogous compounds of AmB provided by the present invention the inventors contemplated the differently proposed action mechanisms of AmB and defined a strategy seeking that the interaction of these drugs with the membrane was as large as possible, so that their structure variations would cause the derivative to act more effectively. Thus, in the present invention they made substitutions in carboxyl opposite to sugar sterically forcing the latter to have more contact with the membrane. For example, a substitution that worked well was to link a tryptophan in this group. Then a derivative was constructed in which histidine was bound to produce not only a steric repulsion forcing sugar to contact the membrane, but also to provide nitrogenated groups to interact with the membrane. While the latter derivative was successful, it was observed that an application for several days led to the development of collateral toxicity similar to that of AmB. It was concluded that this could be owed to the action of a protease breaking the peptide bond and thus, causing a reversal of the derivative of AmB. To solve this problem this bond was protected with a methyl. As a result these derivatives did not significantly affect the action on the membranes with ergosterol, but they considerably reduced their action on the membranes with cholesterol. One way to determine this was to perform unit channel studies, i.e., the channels that characterize the molecular form in the different membranes, to observe the molecular action of the derivatives. While the relative current produced by AmB in a lecithin membrane with ergosterol compared to the same membrane containing cholesterol is 25% higher (Venegas, *et al*, *supra*), and the action of nystatin in POPC membranes containing ergosterol compared to the same membranes with cholesterol has a much higher selectivity, the variation of 25% is obtained when the drug concentration is 10 µM in the first case, and 30 µM in the second (Recamier K., Bachelor degree thesis, UAM 2008).

For the derivatives with substituent amino acids in carboxyl, for example, tryptophan, the selective toxicity is 100% higher than that of AmB. The same applies to the substitution of histidine with the derivatives showing a great advantage in the molecular action on the lipid membrane. It was found that to an identical concentration of 20 µM for membranes with ergosterol or cholesterol the activity is 1100% higher in the fist ones.

Contemplating the model of the formation of AmB channels on the membrane, being those channels responsible for antifungal activity, it is proposed that the relative stability of those channels is mainly owed to three factors that significantly affect the polar head of the molecule, which are: (1) the steric effect; (2) the electronic interactions of the hydrogen bridge; and (3) the changes in the hydrophilic character of the derivative.

Thus, as already adverted to, the design of analogues has focused on the replacement of the carboxyl group, which is exposed to the extracellular medium; this substitution changes the interaction of the drug with the polar heads of the lipids. Contemplating these aspects, a synthesis of a series of AmB analogues was performed as these have the advantage of being produced by a specific reaction of the carboxyl function, which is in the polar head of the molecule and is the region of interest. This ensures that the rest of the molecule remains without structural changes.

Existent AmB derivatives. First, with a brief overview of the state of the art, numerous derivatives of amphotericin B have been reported in literature. These were synthesized to improve their biological activity; therefore, essential information is their minimum inhibitory concentration (MIC), which measures the least amount of drug used to inhibit a fungal colony. The table 1a summarizes the known derivatives of amphotericin B, their main characteristics and changes in their activity resulting from structural modifications.

**Table 1a. Known derivatives of Amphotericin B.**

| **Type of derivate** | **Characteristics** | **Fungicidal activity** | **Modification to the structure** |
|---|---|---|---|
| *Dimer by link bisamine* Matsumori et al [i] | Crosslinking by union of the amino group of carbon 44. | Not reported | 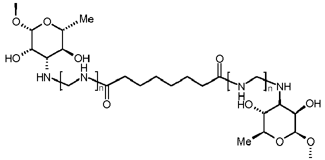 |
| *Dimer by link bisamine* Yamaji et al [ii] | Crosslinking by formation of amides at carboxylic acid of carbon 41 | CIM 0.25 µM^{A} | 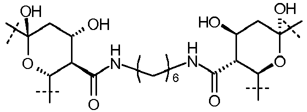 |
| *Conjugated with ergosterol* Matsumori et al. | Conjugation to ergosterol by a hexamethylene carbamate | CIM 20 µM^{B} | 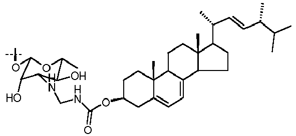 |
| *Without exocyclic carbons* Carmody et al [iii] | Suppression of the exocyclic carboxyl group by removing the gene "*amphN* cytochrome P450" | CIM 5.0 µM^{A} | 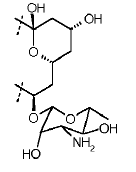 |
| *Bis alkylation of mycosamine* Paquet et al [iv] | Double reductive alkylation on the mycosamine of carbon 44 | CIM 0.02 µM^{A} | 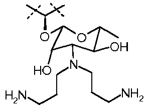 |
| *Conjugated con calixarenes* Paquet et al[v] | Conjugation of four molecules of amphotericine to B-calix[4]arenes | CIM 0.10 µM^{A} | 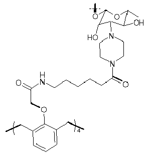 |
| *Aliphatic amides* Morales [3], Jarsebski et al [vi] | Aliphatic amides were formed in the carboxyl group of carbon 41 | CIM 0.323 µM^{A} | 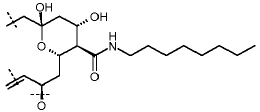 |
| *Aromatic amides* Morales [3] | Aromatic amides were formed in the carboxyl group of carbon 41 | CIM 1.0 µM Martinez [vii]^{A} | 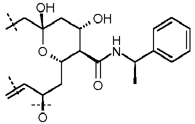 |
| *Conjugated with arabinogalactane* Ehrenfreund-Kleinmana [viii] | Arabinogalactane conjugation in the mycosamine of carbon 44 | CIM 0.25 µM^{A} | 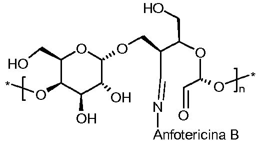 |
| *Conjugated with polyethylene glycol* Conover et al [ix] | Polyethylene glycol conjugation in the mycosamine of carbon 44 | CIM 4.0 µM^{A} | 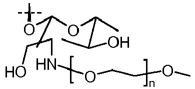 |

| | | | |
|---|---|---|---|
| ^{A.} Activity measurement against *Saccharomyces cerevisae.* ^{B.} Activity measurement against *Candida Albicans.* **[i]** Matsumori, N.; Yamaji, N.; Matsuoka, S.; Oishi, T.; Murata, M. "Amphotericin B Covalent Dimers Forming Sterol-Dependent Ion-Permeable Membrane Channels" J. Am. Chem. Soc. 2002, 124, 4180-4181. **[ii]** Yamaji, N.; Matsumiri, N.; Matsuoka, S.; Oishi, T.; Murata, M. "Amphotericin B Dimers with Bisamide Linkage Bearing Powerful Membrane-Permeabilizing Activity" Org. Lett. 2002, 4(12), 2087-2089. **[iii]** Carmody, M.; Murphy, B.; Byrne, B.; Power, P.; Rai, D.; Rawlings, B.; Caffrey, P. "Biosynthesis of Amphotericin Derivatives Lacking Exocyclic Carboxyl Groups" J. Biol. Chem. 2005, 280(41), 34420-34426. **[iv]** Paquet, V.; Carreira, E.M. "Significant Improvement of Antifungal Activity of Polyene Macrolides by Bisalkylation of the Mycosamine" Org. Lett. 2006, 8(9), 1807-1809. [v] Paquet, V.; Zumbuehl, A; Carreira, E. "Biologically Active Amphotericin B-Calix[4]arene Conjugates" Bioconjugate Chem. 2006, 17, 1460-1463. [vi] Jarzebski, A.; Falkowski, L.; Borowski, E. "Synthesis and structure-Activity Relationships for Amides of Amphotericin B" J. Antibiot. 1982, 35(2), 220-229. **[vii]** Ocampo Martinez, L. "Evaluación toxicológica comparativa de la anfotericina B y sus análogos sintéticos en modelos experimentales in vitro e in vivo" Tesis de Licenciatura. Universidad Autónoma del Estado de Morelos 2007. **[viii]** Ehrenfreund-Kleinmana, T.; Azzama, T.; Falkb, R.; Polacheckb, I.; Golenserc, J.; Domb A. J. "Synthesis and characterization of novel water soluble amphotericin B-arabinogalactan conjugates" Biomaterials 2002, 23, 1327-1335. **[ix]** Conover, C.; Zhao, H.; Longley, C.; Shum, K.; Greenwald R. "Utility of Poly(ethylene glycol) Conjugation To Create Prodrugs of Amphotericin B" Bioconjugate Chem. 2003, 14, 661-666. | | | |

AmB derivatives generated by the present invention. First, it should be pointed out that it has been observed that raising the temperature of AmB above 70°C and exposing the drug to light provokes the loss of its antibiotic action. However, no studies have been made that describe the structural change produced by these two factors. This fact is of great importance when performing reactions with AmB: its temperature should not rise above 50°C for safety, and the drug should be protected from light in order not to affect its biological activity.

Contemplating (1) the steric effect, (2) the electronic interactions of hydrogen bridge, *π*-*π, H*-*π*, and repulsion, and (3) the changes in the hydrophilic character of the derivative to be procured; the synthesis of amide-type derivatives of AmB as these have the advantage of being produced by a specific reaction of the carboxylated function, which is at the polar head of the molecule and which is the region of interest. This ensures that the rest of the molecule will not undergo structural changes. As adverted to above, the choice of amines used to synthesize the derivatives was performed according to the criterion that the resulting amide should provoke said interactions in a favorable way to increase the channel stability and thereby optimizing the antibiotic activity.

The design of the synthesis of derivatives provided by the present invention is done considering the following changes and their behavior in accordance with the proposed effects:
- Structural changes resulting from the change of the carboxyl function to the amide function.
- Conformational changes resulting from the different types of interactions generated by the introduction of each amide group.
- Changes in the stereochemistry resulting from the introduction of new stereogenic centers.
- Changes induction in the behavior of another region of the molecule, which is related with the three previous modifications.

The importance of the steric effect is based on the proposal made by Resat (Resat, et al., J. of Computer-Aided Molecular Design 2000, 14, 689-703) where this effect is treated as a phenomenon of repulsion owed to the amide substituent size of a molecule and the amino carbohydrate unit of the neighboring molecule. Resat proposes that this effect should cause a change in the amide structure, which would lead to further stabilization of the channel structure because of less flexibility in the polyene chain. The polyene chain flexibility is associated with the possibility that the amino carbohydrate unit takes different conformations for the amide group. The different conformations are stabilized by the formation of hydrogen bridges between the amide and carbohydrate groups. Deliberating the idea of Resat, in this patent application the inventors propose that a larger flexibility in the polyene chain will allow the amino carbohydrate unit to present various conformations of the amide group, whether of the same molecule or the neighboring molecule (intra- and intermolecular interactions). Because of this, a greater conformational freedom will lead to a destabilization of the channel structure. However, less flexibility in the chain allows only interactions between the amide groups and the carbohydrate of neighboring molecules; that is, intermolecular interactions, which will cause the channel to present a greater stability. The Scheme 1 shows the shape of the intermolecular interaction proposal. The R group of the derivatives depends on the amine used for the synthesis of the amides.

Resat furthermore proposes that the generation of hydrogen bridges between the nitrogen of the amide group of a molecule and the groups -OH of the carbohydrate in the neighboring molecule would help to stabilize the former intermolecular interaction, resulting in a greater stability of the unit channel. Both proposals are based on the results of molecular dynamics simulations on the behavior of the AmB channel.

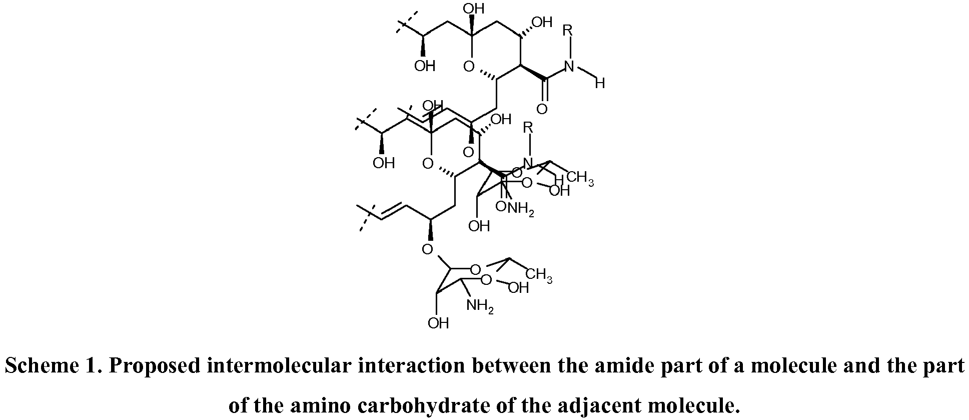

Returning to the idea that intermolecular interactions favor the stability of the unit channel, in this patent application the inventors also propose the existence of electronic interactions between the amide groups of neighboring molecules (Scheme 2). The electronic interactions π-π would occur between the aromatic rings of neighboring molecules in derivates with aromatic substituents (Scheme 3). H-π interactions would occur between the protons of -OH groups in the carbohydrate and the aromatic ring in derivatives with aromatic substituents. The inventors propose that these interactions could lead to a stabilization of the unit channel.

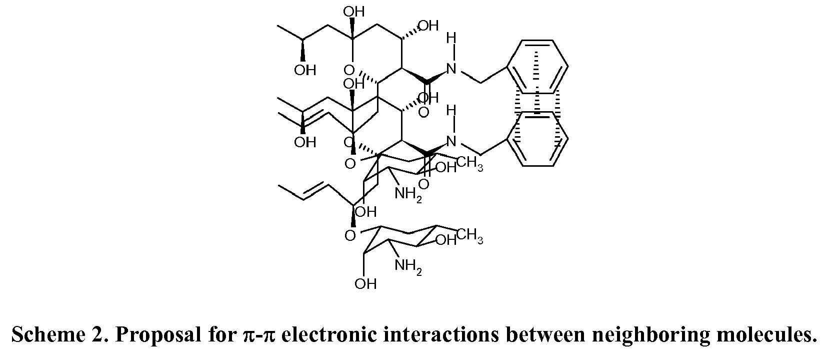

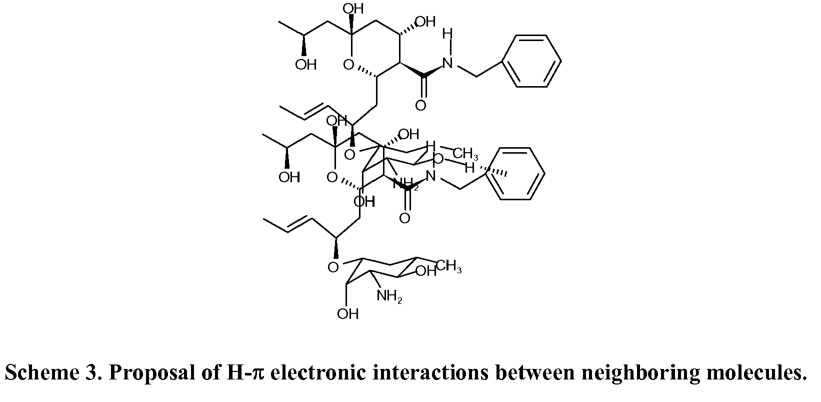

Another proposed effect is the electronic repulsion between the electrons of the -OH groups and the electron density of the aromatic ring in derivatives with aromatic substituents. In this patent application, the inventors propose that this effect could reduce the flexibility in the polyene chain and give greater stability to the channel structure. This effect has some similarity to the steric effect, since it involves the same groups (amide and carbohydrate), but here they propose the repulsion only for the derivatives with aromatic substituents.

Finally, the inventors propose that the hydrophilicity changes in the head of the derivate regarding AmB are important because of the relation (Holtz, et al., J. Gen. Physiol, 1970, 56, 125-145) between the head of the molecule and the hydrophilic part of the lipid membrane for the formation of the unit channel. The use of aliphatic amines for the synthesis of amides decreases the hydrophilic head of the molecule. The decrease of the hydrophilicity of the molecule could generate an unfavorable interaction with the membrane and hence decrease the stability of the unit channel and thus the antibiotic activity.

The synthesis of the amide derivatives of AmB is performed by the method of Preobrazhenskaya (Preobrazhenskaya, et al., J. Med. Chem, 2009, 52, 189-196) or Jarzebski (Jarzebski, et al., J. of Antibio., 1982, 35, 220-229) to procure a series of derivatives spectroscopically characterizable, and that are useful in studying the antibiotic action mechanism of AmB and its amide derivatives, based on their structural modifications. However, it is important to note that in the above work the procuring of amine derivatives of AmB do not primarily intend to destine them for medical practice. There have been previous studies on the biological activity of some derivatives of this type resulting that they have an antibiotic activity similar to AmB, but with the same side effects (Jarzebski, et al., *supra).*

The discussion about a greater or reduced stability of the channels formed by the derivatives will be conducted based on the results of the tests of biological activity in yeast cultures.

A further aspect of the present invention is to provide the synthesized derivatives that support the research related to the mechanism of action of AmB in lipid bilayers by using electro physiological techniques. The present invention contributes thereby to the study of how the derivatives channels are modified regarding those of AmB, and the importance of the changes made in the derivatives.

This method consists of treating 1.0 equivalent (1.0 mmol) of AmB at room temperature in 20 mL of N,N-dimethylacetamide (N,N-DMAc) with 10.0 equivalents (10 mmol) of triethylamine (Et3N), 10 equivalents (10.0 mmol) of the amine needed to make the desired amide and 10 equivalents (10 mmol) of diphenylphosphorylazide, following the course of the reaction by chromatography plate (Scheme 4).

The reaction is carried out using AmB to a ratio of 1:10 reagents to ensure total consumption of AmB by decreasing the likelihood of unreacted AmB, which facilitates the purification of the procured product. AmB is a very polar molecule because of the presence of 10 -OH groups, the amino carbohydrate ring and the acid function, which difficult its dissolution in common organic solvents such as THF, hexane of ethyl ether. The solvent used is N,N-DMAc because AmB has a relatively favorable solubility in this solvent. The Et₃N is used for the generation of AmB carboxylate and to neutralize acid species produced during the reaction. The diphenylphosphorylazide is used to activate the -OH group of the carboxylate as a good salient group which favors the nucleophilic addition of the amine to generate the amide. This amine should be preferably primary, or less favorably secondary to facilitate the reaction, since the reactivity of primary amines to this reaction is greater than the reactivity of secondary amines. Scheme 5 shows the proposed reaction mechanism for the synthesis of such derivatives.

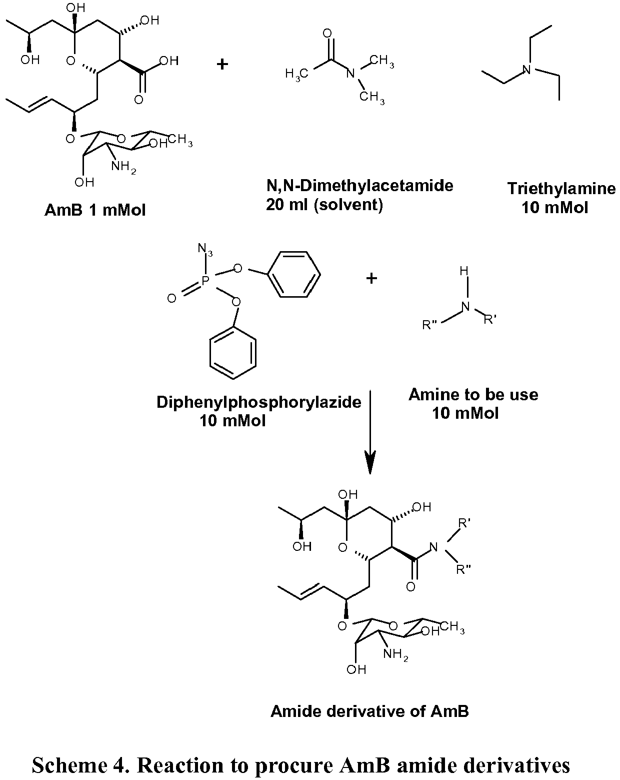

In the first step of the mechanism, Et₃N is the base that abstracts the proton of the carboxylic acid of AmB (**A**) to generate the carboxylate (**B**). This carboxylate is in turn nucleophilically aggregated to diphenylphosphorylazide to generate the phosphonic anhydride (**C**) in which the acid -OH group has been activated as a salient group. On this intermediate (**C**) the nucleophilic amine performs the addition-elimination process, which essentially generates the protonated amide (**D**). Finally, the dyphenylphosphonic anion abstracts the proton of the nitrogenated function to yield the neutral amide derivative of AmB.

The choice of the amines used to synthesize the derivatives is done considering that the resulting amide should provoke a steric effect and electronic interactions with the amino carbohydrate unit among neighboring molecules. Additionally, the changes in the hydrophilicity of the amide should be contemplated regarding the AmB; this, owed to the interaction of the AmB head and the polar part of the membrane. From the generation of these effects and the results of biological tests, the inventors propose a greater or reduced structure stability of the channel formed.

According to especially preferred embodiments of the present invention and without this resulting in a limitation of it scope, the following amines were used in the synthesis exclusively as an example thereof: (1) benzylamine, (2) cyclohexylamine, (3) diisopropylamine, (4) (*S*)-α-phenylethylamine, (5) (*R*)-α-phenylethylamine, (6) L-tryptophan, y (7) D- tryptophan to procure the corresponding amides.

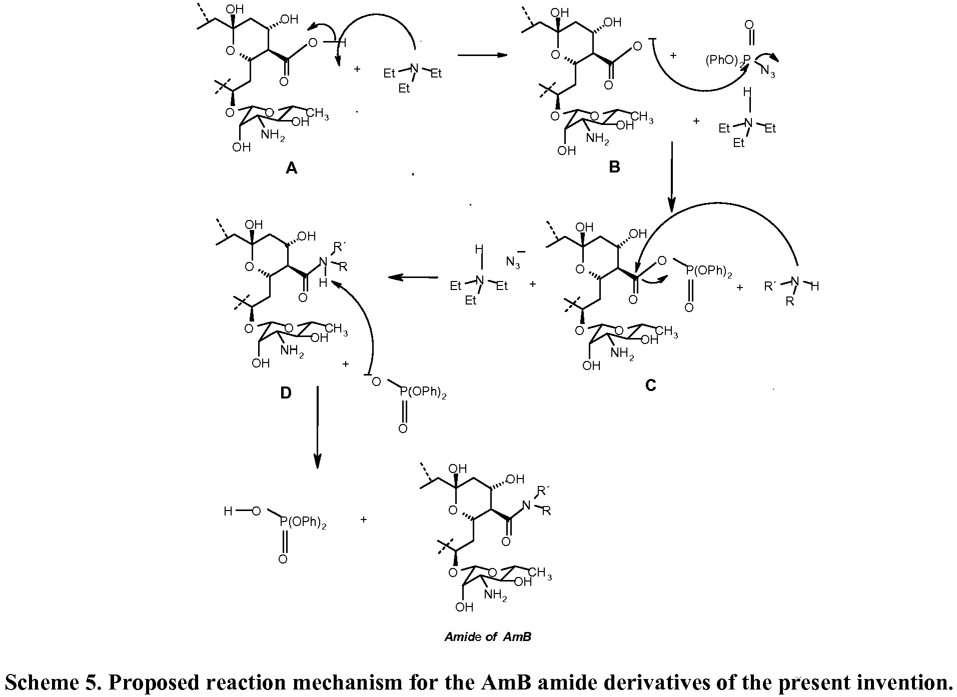

**Analogues examples and chemical synthesis.** The examples presented below are for illustration only and should not be interpreted as a limitation, since the scope of the present invention is limited only by the appended claims.

As adverted to before, the synthesis reaction to procure the amide derivatives of the present invention was made according to the method delineated by Jarzebski (Jarzebski, *et al., supra),* which roughly speaking consists of treating at room temperature 1.0 equivalent (1.00 mmol) of AmB in 20.0 mL of N,N-dimethylacetamide (N,N-DMAc) with 10.0 equivalents (10.0 mmol) of triethylamine (Et₃N), 10.0 equivalents (10.0 mmol) of the amine needed to produce the desired amide and 10.0 equivalents (10.0 mmol) of diphenylphosphorylazide, following the course of the reaction by chromatography plate (Scheme 4). Following the mentioned synthesis methodology, succeeding derivatives were prepared:
**Example 1. Synthesis of amide 1: N-benzylamide of AmB.** A preferred embodiment of the present invention provides the analogue of AmB denominated amide 1: N-benzylamide of AmB, represented by formula I; using benzylamine as the starting amine.

The effects obtained with this derivative are:
- Steric effect between the aromatic ring and the amino carbohydrate unit.
- Hydrogen bridges between the amide nitrogen and the carbohydrate -OH groups of the neighboring molecule, besides the generation of a hydrogen bridge between the amide hydrogen and the -OH group at the β position to the carbonyl. This interaction is strong because of the possible formation of a 6-membered ring stabilized by the partial character of double bond between the nitrogen and the carbonyl carbon. This bond could be generated in all amides with one hydrogen in the amide group. 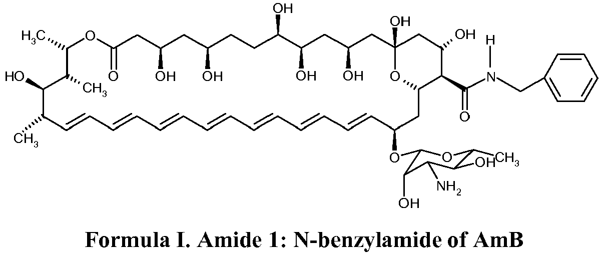
- π-π interactions between the aromatic rings of neighboring molecules, and H-π interactions between the hydrogens of the -OH groups in the carbohydrate and the aromatic ring.
- The reduction of the polar character in the head of the derivative.

It is proposed that the first three interactions could result in a channel stabilization of the derivative. The latter would have a destabilizing effect owed to a lower affinity of the head of the derivative with the polar part of the lipid membrane.

Characterization of amide N-benzylamide of AmB. In the IR spectrum of the product the inventors found the broad band characteristic of the OH vibration, which, because only the OH group of carboxylic acid was changed, is almost identical to that of AmB. They also observed the disappearance of the acid carbonyl band at 1711.0 cm⁻¹ and the appearance of that of amide carbonyl at 1645.4 cm⁻¹. In addition, there is the presence of the characteristic vibration of the aromatic ring at 1605.1 cm⁻¹. The derivative had a value of Rf = 0.75 for what it is contemplated that qualitatively this product is non-polar regarding AmB. According to the proposal of Resat, the generation of the proposed steric effect and the hydrogen intramolecular bridges should be contemplated as the most important interactions that favor the formation of the channel of the derivative. Therefore, it is expected that the antibiotic activity of the derivative is not much different of that of AmB. The inventors propose that the intramolecular hydrogen bridge formed by the 6-membered ring on the head of the derivative may diminish the conformational freedom in that part of the molecule and thus, give more stability to the channel of the derivative. As adverted to above, this interaction could occur in all the derivatives with one hydrogen on the nitrogen of the amide.

**Example 2. Synthesis of the amide 2: N-cycloheximide of AmB.** In another preferred embodiment the present invention provides the analogue of AmB denominated amide 2: N-cycloheximide, represented by formula II; using cycloheximide as the starting amine.

For this synthesis the inventors contemplated only two aspects: the steric effect between the cyclohexil ring and the carbohydrate; and the decrease in the hydrophilic character of the derivative.

The first aspect would lead to stabilization in the channel structure, while the second aspect would produce a destabilization.

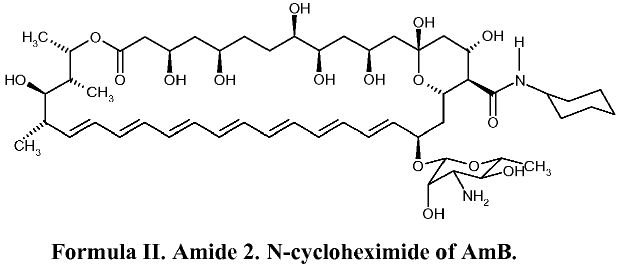

Characterization of amide N-cycloheximide of AmB. In analyzing the IR spectrum of this product, it was found that the band corresponding to the OH vibration of the polyhydroxylated chain was much smaller than that of AmB. This led to assume that the product (amide 2a) showed a solvation effect with dimethylacetamide in the OH groups of the polyhydroxy chain. However, it is also contemplated that if this happens, the band of the OH groups should also have a slight spectrum shift to lower frequencies (red shift), which is hardly noticeable in the spectrum.

Assuming the solvation of the product, this was dried under reduced pressure. The IR spectrum of the product subjected to this treatment (amide **2b**) shows a significant augment in the band of the polyhydroxilated chain, but without achieving the intensity of AmB. Since the models for the formation of the unit channel the polyhydroxilated chains favor the exit of K+ ions, the decrease of the polarity in this region presumably results in less effectiveness regarding the antibiotic activity of the solvated amide (amide **2a**). It is noteworthy that the rest of the spectra of both products are the same, having also the disappearance of the acid carbonyl band at 1711.0 cm⁻¹ and the appearance of the amide carbonyl band at 1640.9 cm⁻¹. Here, both products had a value of Rf = 0.81 for what it is qualitatively that the products are non-polar regarding AmB.

Another important factor would be the possibility that the antibiotic behavior is diminished because the actual concentration of the derivative would be less than that contemplated in the preparation of the amide solution for the determination of the antibiotic activity.

The generation of the steric effect and the intramolecular hydrogen bridges is contemplated as the most important interactions, favoring the formation of the channel of the derivative. Therefore, it is contemplated that the antibiotic activity of the derivative should not be very different to that of AmB. However, because of the great decrease showed in the polar character of the head of the derivative in this case, this would be an effect that would decrease the channel stability and thus, the antibiotic activity. According to the results of tests on yeast cultures, the priority of the effects adverted to may be proposed.

**Example 3. Amide 3 synthesis: N-diisopropylamide of AmB.** In another preferred embodiment the present invention provides the analogue of AmB denominated amide **3**, N-diisopropylamide of AmB, represented by formula III, using diisopropylamine as the starting amine:

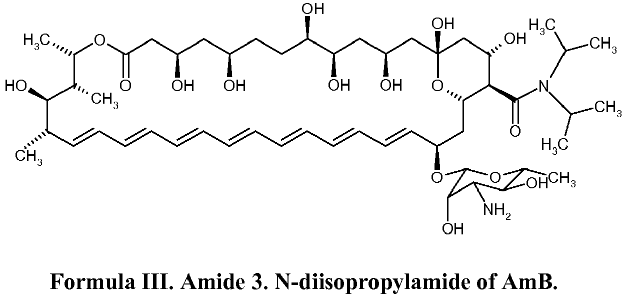

For this synthesis the invention contemplates two aspects: a strong steric effect between the two isopropyl groups and the carbohydrate, and the elimination of the hydrophilic head of the derivate.

The first aspect would lead to stabilization in the channel structure, while the second aspect would create a destabilization.

Characterization of amide N-diisopropylamide of AmB. It was found in the IR spectrum of the derivative that the characteristic band of the polyhydroxylated chain was almost the same as that of AmB. The inventors observed the disappearance of the acid carbonyl band at 1711.0 cm⁻¹ and the appearance of the amide carbonyl at 1642.8 cm⁻¹. The derivative had a value of Rf = 0.7, and it is therefore considered qualitatively that this product is non-polar regarding AmB.

For this derivative, contemplating the two effects, the steric and the decrease of polarity are considered very important. Because of the increased conformational freedom of the isopropyl group it is proposed that the steric effect may be greater than necessary for the formation of intermolecular hydrogen bridges and thus promote the stability of the unit channel, and thus have a lower antibiotic activity than AmB. In addition, resulting from the decrease in polarity, it is possible to deliberate that this would further diminish the antibiotic activity owed to a lower stability of the unit channel.

**Example 4. Synthesis of amide 4: N-(S)-α-phenylethylamide of AmB.** In another preferred embodiment the present invention provides the analogue of AmB denominated amide 4: N-*(S)-α-*phenylethylamide of AmB, represented by formula IV; using *(S)-α-* phenylethylamine as the starting amine.

The synthesis of this derivative was chosen considering following aspects:
- The steric effect owed to the aromatic ring and the methyl group with the carbohydrate. This effect is even greater than that of the amide **1**.
- A possible formation of hydrogen bridges between the -OH groups of the carbohydrate with the amide group.
- The generation of π-π interactions between the aromatic rings of the neighboring molecules and the H-π interactions between the hydrogens of the -OH groups with the aromatic ring. The repulsion between the electron density of the aromatic ring and the electrons of the -OH groups of carbohydrate.
- The significant decrease in the polar character of the derivative head owed both to the presence of the aromatic ring and the methyl group that is in the position α to it.

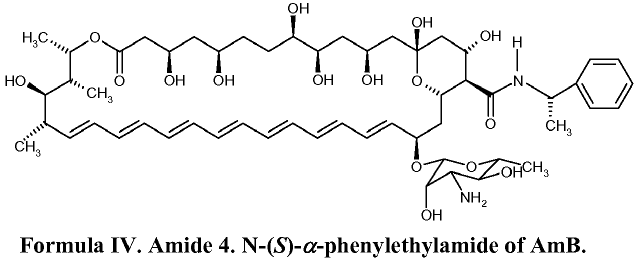

The first three interactions could lead to a stabilization of the derivative channel. However, this effect is more difficult to predict because the steric effect may be greater than necessary to produce a favorable interaction. However, the latter aspect would have a destabilizing effect by generating an unfavorable hydrophilic interaction.

Characterization of amide N-(S)-α-phenylethylamide of AmB. The characteristic band of the OH vibration was found in the IR spectrum of the product, and it is almost identical to that of AmB. In addition, the disappearance of the acid carbonyl band was observed at 1711.0 cm⁻¹ and the appearance of the corresponding amide carbonyl band at 1631.3 cm⁻¹. Finally, there is the presence of the characteristic vibration of the aromatic ring at 1612.0 cm⁻¹. The derivative had a value of Rf = 0.73 for which it is qualitatively contemplated that this product is non-polar regarding AmB.

For this derivate the factor with a higher priority is the steric effect, which is higher than for the amide 1 owed to the presence of the methyl group, which may further reduce the conformational freedom, thus, favoring the formation of intermolecular hydrogen bridges would lead to greater stability of the unit channel and therefore, in an analogous antibiotic activity or maybe higher than that of AmB. However, the steric effect could be greater than necessary to elicit a favorable interaction, which would decrease the antibiotic activity. This could also determine the importance of the methyl group in the structure and the difference in behavior regarding the amide 1.

The second most important effect would probably be the formation of π-π interactions between the aromatic rings of the neighboring molecules and H-π interactions between the hydrogens of the -OH groups with the aromatic ring, which would favor the stability of the unit channel and thus, have an augment in the antibiotic activity similar to that of AmB.

According to the proposal of Resat, the decrease of the polarity in the derivative head would be the effect of lower priority. This supports the idea that the derivative could have an antibiotic activity similar to that of AmB.

**Example 5. Synthesis of amide 5: N-(R)-α-phenylethylamide of AmB.** In another preferred embodiment the present invention provides the analogue of AmB denominated amide 5: N-(R)-α-phenylethylamine of AmB, represented by formula V, using *(R)*-*α*-phenylethylamine as the starting amine.

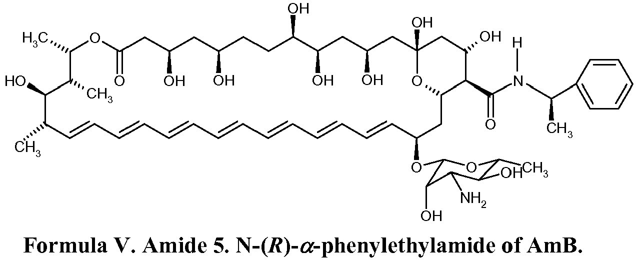

In the synthesis of this amide the antibiotic effect was observed compared to the amide **4** owed to the spatial orientation of the aromatic ring provided by the stereogenic center; the effect of the presence of the methyl group regarding the amide **1** was also observed.

Characterization of the amide N-(R)-α- phenylethylamide of AmB. The IR spectrum of the product is almost identical to that of amide **4**, with the amide carbonyl band at 1630.1 cm⁻¹, showing only slight differences in the intensity of the signals._The derivative had a value of Rf = 0.73 for which it is qualitatively contemplated that this product is non-polar regarding AmB. These two results help to confirm that the product has the same structure of amide **4**, and its epimer.

Here, being the epimer (R) of amide **4**, the same effects of those of derivative **4** are considered. Therefore, this synthesis is to determine whether the difference in the stereochemistry of the epimers (S) - (R) will have any effect on the antibiotic activity.

**Example 6. Synthesis of the amide 6: N-(*L*)-tryptophanamide of AmB.** In another preferred embodiment the present invention provides the analogue of AmB denominated amide 6: N-(L)-tryptophanamide of AmB, represented by formula VI; using L-tryptophan as the starting amine.

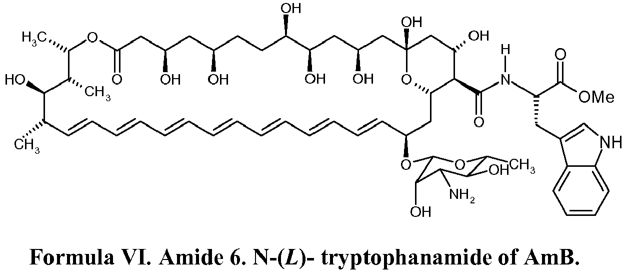

This amide was synthesized considering the following aspects:
- To provide amide derivatives that are UV fluorescent to form channels in membranes and susceptible to UV spectroscopy.
- The heterocyclic structure of indole and the methylene group they contain cause a steric effect with the carbohydrate unit. This effect is greater than in the previous amides.
- A possible generation of hydrogen bridges between the -OH groups in the carbohydrate with the amide group.
- The electron delocalization of the indole ring generates aromaticity, which could encourage π-π interactions between the indole rings of the neighboring molecules. There is repulsion between the electronic density of the indole ring and the electron pairs of the -OH groups of the carbohydrate.
- The decrease in the hydrophilic head of the derivative (lower than in previous derivatives) owed to the presence of the indole ring, the methylene group and the methylic ester.

Characterization of amide N-*(L)*-tryptophanamide of AmB. First, the protective reaction of the acid group of L-tryptophan was performed in the form of methyl ester hydrochloride of the L-tryptophan. The ester characterization was performed by gas mass chromatography and determination of melting point.

In the IR spectrum was observed the characteristic band of the OH vibration, which is almost identical to that of AmB. Additionally, the disappearance of the acid carbonyl band was observed at 1711.0 cm⁻¹ and the appearance of the amide carbonyl band at 1635.4 cm⁻¹. The derivative had a value of Rf = 0.62 for which it is qualitatively considered that this product is low polar regarding AmB.

Given the presence of the indole and methylene groups, the steric effect may be excessive for the formation of intermolecular hydrogen bridges; it is therefore proposed here that the result would be a decrease in antibiotic activity resulting from a decreased stability of the unit channel. It is also suggested that because of the electron delocalization present in the indole ring, π-π interactions would be produced that would give greater stability to the unit channel generating a greater antibiotic activity. Moreover, here the polarity decrease is not as large as in the previous amides so that this destabilization interaction of the channel is not important. However owed to the diverse nature of the factors involved in this derivative, it is complicated to predict the priority of the above effects on its antibiotic behavior.

One of the main objectives of the synthesis of amide 6 and its epimer, amide 7, is to provide AmB derivatives substituted by the metoxy-tryptophanamine group, whose channels are supposed to present UV fluorescence.

**Example 7. Synthesis of amide 7: N-(D)-tryptophanamide of AmB.** In another preferred embodiment the invention provides the AmB analogue denominated amide **7**: N-*(D)*-tryptophanamide of AmB, represented by formula VII; using *D*-tryptophan as the starting amine.

Fort this derivative the same structural aspects of the synthesis of amide **6** were taken into account and it was observed in its synthesis the possible effect on the antibiotic activity that has the change in the stereochemistry of this derivative regarding amide **6.** For L-tryptophan and D-tryptophan, before the amide synthesis, it was necessary to perform the sterification reaction of the carboxylic acid to protect it in form of hydrochloride of the methyl ester of tryptophan.

This synthesis was performed by reacting 1 equivalent of the amino acid L-tryptophan or *D*- tryptophan (*L*-Trp or *D*-Trp) with excess MeOH and 2 equivalents of Me3SiCl to procure the white precipitate of the hydrochloride of the methyl ester of L-tryptophan or *D*- tryptophan. Scheme 6 shows the reaction mechanism proposed for this synthesis.

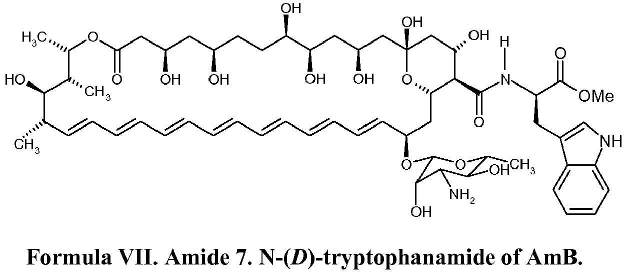

The first step in the reaction consists of the nucleophilic addition of the tryptophan carboxyl function (**E**) on the Si of Me₃SiCl (**F**) inducing the displacement of the Cl⁻ ion. The silicon esters (intermediates **G** and **H**) are in an equilibrium in which it is proposed that the deprotonated form is more susceptible to the nucleophilic addition of MeOH. This will form the hydrochloride of the methyl ester tryptophan with trymethylsilanol as a byproduct of the reaction. The hydrochloride of the tryptophan methyl ester is used for the synthesis of the amides of AmB incorporating only an excess of Et₃N as basic reagent in the method of Jarzebski. This liberates the form of the methyl ester of the tryptophan to act as amine in the reaction.

It is reported that by incorporating the tryptophan structure in AmB polyene molecules they fluoresce under UV light. The tryptophan methyl ester was used for the synthesis of AmB amides as a means to procure amides that fluoresce under UV light. This is a desirable characteristic for experiments of electrophysiology in unit channel as because of this, the channels would present this type of fluorescence.

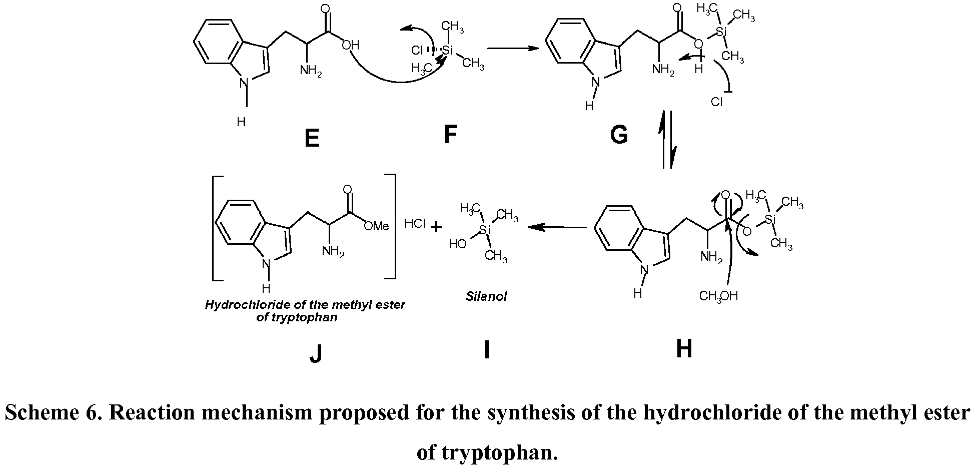

Characterization of amide N-(D)-tryptophanamide of AmB. The IR spectrum of the product is almost identical to that of amide 6, showing only slight differences in the intensity of the signals. The derivative had a value of *R_{f}* = *0.62* for which it is qualitatively considered that this product is non-polar regarding AmB. These two results help confirm that amide **7** has the same structure of amide 6, and its epimer.

During the process of purification of amide **7** two products were isolated: amide **7a** and amide **7b**. The IR spectra of both amides have a similar difference to that of amide **2a** and amide **2b** in the absorption band of the polyhydroxylated chain. Here the difference in the intensity of the bands is not too large. Because of this it is believed that this is the same structure, and it is supposed that the solvation with ether (of the purification) occurred in the polyhydroxylated chain for the amide **7b**.

Moreover, it is likely that the antibiotic behavior is diminished because the actual concentration of the derivative could be less than that contemplated in the preparation of the amide solution. It is noteworthy that the rest of the spectra of both products are the same. Here, being the epimer (*D*) of the N-(*L*)-tryptophanamide, the same values of the derivative **6** are contemplated. Therefore, this synthesis is to determine whether the difference the epimers *(L)* - *(D)* stereochemistry will affect the antibiotic activity.

**Example 8. Synthesis of amide 8: N-histaminamide of AmB.** In another preferred embodiment the present invention provides the analogue of AmB denominated amide 8: N-Histaminamide of Amb, represented by formula VIII, using amphotericin B, N,N-dimethylacetamide, triethylamine, histamine and diphenizphosphorylazide as starting materials. In a 100 mL flask ball wrapped in foil to avoid light, 462 mg (0.5 mmol) of amphotericin B were weighed and dissolved in 10.0 mL of N,N-dymethylacetamide under nitrogen atmosphere. Then 0.7 mL (5.0 mmol) of triethylamine, 5.0 mmol of histamine, and 1.08 mL (5.0 mmol) of diphenizphosphorylazide were added.

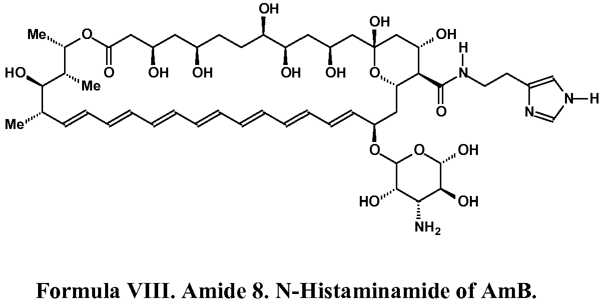

The reaction was left at room temperature with constant stirring for a period of 12 hours. The progress of the reaction was measured by thin layer chromatography in the system chloroform:methanol:water (20:10:1). Subsequently, the product of the reaction was precipitated by adding 150 mL of anhydrous ethyl ether and let stand until the precipitation was completed, which is normally associated with the clearance of the ether solution. Ethyl ether was decanted and the formed precipitate was dissolved in 1-butanol and washed twice with 50 mL of distilled water. Subsequently the 1-butanol was evaporated under reduced pressure (10 mmHg) at 25°C; the derivative was precipitated with 50 mL ethyl ether and washed three times with 50 mL ethyl ether and 50 mL hexane. The product was vacuum dried. Finally, the compound 8 was obtained with a 92% yield after product purification.

**Example 9. Synthesis of amide 9: (3-pyridylamide) of AmB.** In another preferred embodiment the present invention provides the analogue of AmB denominated amide 9, represented by formula IX; using amphotericin B, N,N-dimethylacetamide, triethylamine, 3- aminomethylpyridine and diphenizphosphorylazide as starting materials.

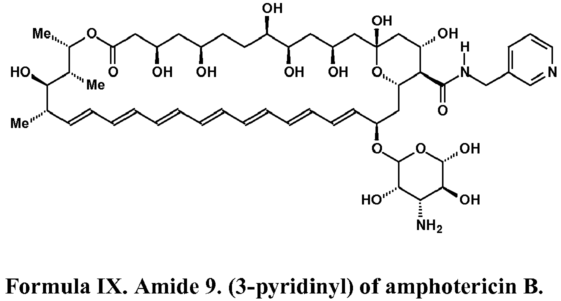

In a 100 mL flask ball 462 mg (0.5 mmol) amphotericin B were weighed and dissolved in 10 mL de N,N-dimethyl acetamide. The flask was wrapped in aluminum foil as amphotericin B decays in the presence of sunlight; the reaction was performed under nitrogen atmosphere. Subsequently 0.7 mL (5.0 mmol) of triethylamine, 5.0 mmol of 3-aminomethylpyridine and 1.08 mL (5.0 mmol) of diphenizphosphorylazide were added. The reaction was left at room temperature with constant stirring until disappearance of the raw material (amphotericin B). The progress of the reaction was measured by thin layer chromatography in the system chloroform:methanol:water (20:10:1). Subsequently, the reaction was precipitated in 150 mL of anhydrous ethyl ether and let to stand until the product precipitated completely, which is normally associated with the clearance of the ether solution. Ethyl ether was decanted and the precipitate formed was dissolved in 1-butanol and washed twice with 50mL of distilled water. Subsequently, 1-butanol was evaporated under reduced pressure (10 mmHg) at 25°C. Finally, the derivative was precipitated with 50 mL of ethyl ether and washed three times with 50 mL of ethyl ether and once with 50 mL of hexane. The product was vacuum dried. In this reaction, the analogue **9** was obtained with a 95.09% yield after product purification.

**Example 10. Synthesis of amide 10 (derivative A21): N-(L)-Histidinamide of AmB.** In another preferred embodiment the present invention provides the analogue of AmB denominated amide 10 or derivative A21: N-(*L*)-Histidinamide of AmB, represented by formula X, and prepared according to reaction scheme 7 based on the quantity of reagents showed in table 1b.

For this synthesis reaction the compounds required for each equivalent of Amphotericin B are: 10.0 equivalents of (Et)₃N, 10.0 equivalents of diphenylphosphorylazide, and 10.0 equivalents of the amine to be used, depending on the amide desired.

In a 3-necked flask of 100 mL 0.250 g of AmB were dissolved in 5.0 mL N,N-DMAc; the inert atmosphere was procured by injecting nitrogen to the reaction flask; 0.38 mL of (Et)₃N (triethylamine), 0.652 g of dihydrochloride of methyl ester (*L*)-histidine, and 0.58 mL of diphenylphosphorylazide were added. The progress of the reaction was monitored by thin layer chromatography on silica gel in a chloroform:methanol:water (20:10:1) system. After 72 hours of reaction the reaction product is precipitated with 50 mL of ethyl ether and refrigerated for 12 hours; the ether was decanted, and the resulting product was dissolved in the least amount of 1-butanol. Then the product was washed twice with 100 mL of water. The butanol-water azeotrope was distilled then under reduced pressure (10 mmHg) at 50°C controlled by oil bath and gentle shaking. After distillation the product was precipitated again with 50 mL of ethyl ether and left in the refrigerator for 12 hours, and the ether was decanted. The product was washed three times with 50 mL of ether, one with 50 mL hexane, and vacuum dried.

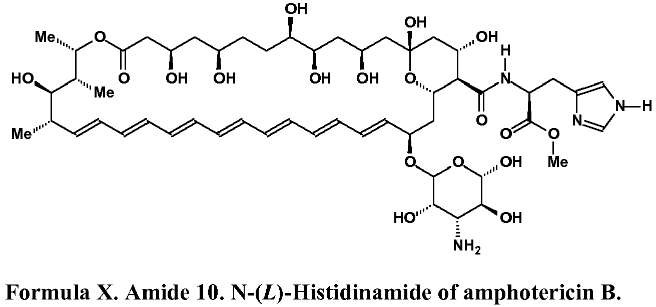

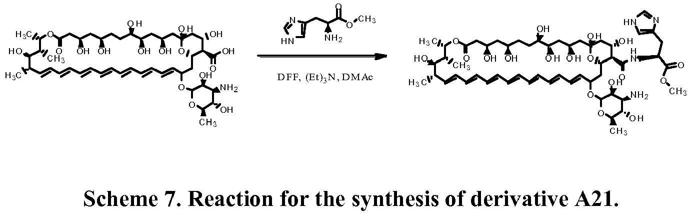

**Table 1b. Quantities used in the preparation of A21.**

| **Substance** | **M (g/mol)** | **Density (g/mL)** | **m (g)** | **V (mL)** | **n (mol)** |
|---|---|---|---|---|---|
| AmB | 924 | | 0.250 | | 2.7 E -4 |
| (Et)₃N | 101.19 | 0.726 | 0.274 | 0.38 | 2.7 E -3 |
| Methyl ester of L-Histidine 2 HCl | 241 | | 0.652 | | 2.7 E -3 |
| Diphenylphosphorylazide | 275.2 | 1.273 | 0.7445 | 0.58 | 2.7 E -3 |

Observations on AmB and its amide derivatives of the invention. AmB and its derivatives have a very high solubility in DMSO and *n*-BuOH, measuring the concentrations up to 2 x 10⁻² M for DMSO. Its solubility in water is much lower. The course of the synthesis reactions for amides was followed by thin layer chromatography. The determination of the retention factors (*R_{f}*) of each amide was used as a qualitative measure of changes in the polarity of the derivatives regarding AmB. The *R_{f}* values were obtained in the same solvent system, chloroform:methanol:water 20:10:1 (V/ V/ V) for AmB and its derivatives. Since for this system AmB remains at the point of application, the value of its *R_{f}* = 0. Therefore, the derivatives with an *R_{f}* value close to the unit they are contemplated as non-polar regarding AmB, whereas for derivatives with an *R_{f}* value further from the unit they are contemplated of low-polarity regarding AmB. All amides were characterized by IR spectroscopy to detect the vibrations of characteristic groups of AmB and its derivatives.

Because of the structures of the derivatives the spectra are very complex, so only the most important vibrations have been specified. The regions in which these vibrations are found are (Rubinson, et al., Contemporary Chemical Analysis, Prentice Hall, 1998, 352-364, 380-403):
- 3500 - 3200 cm⁻¹ (f), polyhydroxylated chain stretching.
- 2990 - 2850cm⁻¹ (m-f) CH₃ and CH₂ stretching in aliphatic compounds.
- 1725 - 1700 cm⁻¹ (m) acid carbonyl stretching.
- 1680 - 1630 cm⁻¹ (m) amide carbonyl stretching.
- 1615 - 1590 cm⁻¹ (m) benzene ring.

The appearance of a band in the amide carbonyl region and the disappearance of the acid carbonyl band of AmB is a useful characteristic in determining the structure of amide derivatives. The IR spectrum of AmB shows a very strong and broad signal in the region of 3500 - 3200 cm⁻¹ corresponding to the polyhydroxylated chain. Since no changes happen in this part of the structure, this band should be essentially the same for amide derivatives.

For AmB and its derivatives melting points were taken, but in all cases we observed the decomposition of the samples in a range of 100 to 150°C.

Owed to the low solubility of the derivatives in solvents suitable for NMR analysis and the resource limitations of the equipment for the analysis of molecules of such complexity, it was not possible to procure good spectra of NMR ¹H and NMR ¹³C. However, the spectra procured show carbon and proton signals in the regions of some important groups of the derivatives such as: the presence of vinylic groups (polyene chain), methyl and methylene, aromatic systems (in the derivatives having them), and the anomeric carbon (of the amino carbohydrate).

Mass spectra were performed with FAB⁺ to have a fragmentation pattern that allows the generation and detection of the molecular ion of the derivatives (Rubinson, et al., *supra).*

All derivatives were tested for antibiotic activity in yeast cultures of *S. cerevisiae.*

First, we will discuss the results of the synthesis of amide derivatives and their characterization, and the possible effects of their structure in the unit channel stability. Later we will analyze the results obtained regarding the antibiotic activity showed in yeast cultures. We will do this to rationalize the effects of the substituents regarding the proposal on the unit channel stability.

Amide derivatives of AmB. Table 2 shows a list of seven of the synthesized amides, the structural modifications performed regarding the effects of AmB and the favorable and unfavorable effects to the antibiotic activity according to the changes made. This table will be discussed in detail below.

Comparison of the absorption bands in IR. For all the amide derivatives, it was found that the absorption band of acid carbonyl corresponding to AmB. Instead of this, a band appears in the region of amide carbonyl absorption for the various amide derivatives (Rubinson, et al., *supra*). The absence of the acid carbonyl band and the presence of that of carbonyl amide is a characteristic of the products of the reactions. Table 3 shows the reaction yields obtained for each derivative and the values of the acid carbonyl absorption bands of AmB and of carbonyl amide for the derivatives.

**Table 2. Amides synthesized and their modifications.**

| **Derivative of AmB** | **Structural modifications and polarity regarding AmB** |
|---|---|
| N-benzylamide (amide **1**) | Benzylamine group |
| N-cycloexylamide (amide **2a**) | Cyclohexylamine group |
| N-cycloexylamide (amide **2b**) | Cyclohexylamine group |
| N,N-diisopropylamide (amide **3**) | Diisopropylamine group |
| N-(S)-α-phenylethylamide (amide **4**) | (S)-α-phenylethylamine group |
| N-(R)-α-phenylethylamide (amide **5**) | (R)-α-phenylethylamine group |
| N-(L)-tryptophanamide (amide **6**) | (L)-methoxytryptophanamine group |
| N-(D)-tryptophanamide (amide **7a**) | (D)-methoxytryptophanamine group |
| N-(D)-tryptophanamide (amide **7b**) | (D)-methoxytryptophanamine group |

**Table 3. Reaction yields and absorption bands for AmB and its amide derivatives**

| **Derivative** | **Yield (%)** | **IR absorption bands (cm⁻¹)** | |
|---|---|---|---|
| | | **Acid carbonyl** | **Amide carbonyl** |
| AmB (purification) | 97.15 | 1711.0 | -- |
| N-benzylamide (amide **1**) | 95.50 | -- | 1645.4 |
| N-cycloexylamide (amide **2**) | 88.90 | -- | 1640.9 |
| N,N-diisopropylamide (amide **3**) | 93.11 | -- | 1642.8 |
| N-(*S*)-α-phenylethylamide (amide **4**) | 99.00 | -- | 1631.3 |
| N-(*R*)-α- phenylethylamide (amide **5**) | 98.45 | -- | 1630.1 |
| N-(*L*)-tryptophanamide (amide **6**) | 65.13 | -- | 1635.4 |
| N-(*D*)-tryptophanamide (amide **7**) | 93.85 | -- | 1638.2 |

### Preclinical testing.

Biological assays - Cytotoxicity evaluation. Cytotoxicity refers to any damage cause to cells by chemical, physical, and biological agents, which can range from a morphological and functional alteration to causing death. The evaluation of cytotoxicity can be accomplished by observing various parameters such as cell morphology, cell viability, cell adhesion, cell proliferation, membrane damage (erythrocytes) or metabolic disorders.

For cytotoxicity studies human kidney cells (ATCC No. CRL-1573) were used. The cells were cultured with Minimum Essential Medium (MEM) (GIBCO BRL), supplemented with 10% fetal bovine serum (FBS) (GIBCO BRL), 2 mM l-glutamine (GIBCO BRL), 1.5 gL Na₂HCO₃ (Sigma Chemical Co), 0.1 mM nonessential amino acids (Sigma Chemical Co), and 1 mM sodium pyruvate (In vitro, S.A.). The technique of bromide of 3(4,5-dimethylthiazol-2-yl)-2,5-diphenyl tetrazolium (MTT) (Sigma Chemical Co) was applied; a technique which is based on the conversion of dye to an insoluble precipitate called formazan. This test is used as an indicator of mitochondrial function in living cells that measures the metabolic capacity in cells and serves as an indicator of cytotoxicity. It has been reported that the results of this test are comparable in sensitivity with those obtained using the incorporation of [3H] (Mosman 2000, Inmunol Methods, 65: 55-63). The cytoxicity test consisted of planting 10,000 cells per well in 96-well plates (Corning Incorporated Costar) in MEM and incubate them (Nuaire us autoflow CO₂ water-jacketed incubator) during 24 h at 37°C and 5% CO₂. Eight different treatment levels were included (0.1, 0.2, 0.4, 0.6, 0.8, 1.0, 10 and 100 µM) of the analogues and AmB of the invention; a group treated with DMSO at 1% was also included. After incubation, cells were treated and incubated anew for 24 h. After this time the medium was aspirated carefully and replaced with 200 µL of fresh medium, and 50 µL of MTT was added for a total volume of 250 µL. They were then incubated for 4 hours in the above conditions. After that time the medium with MTT was carefully removed and 200 µL of DMSO and 25 µL of Sorensen buffer (0.1 M glycine + 0.1 M NaCl at pH 10.5) were added to each well. The plate was then mixed until the formazan crystals dissolved, and it was finally read in a plate reader (Microplate Limaning System Ultramark, Biorad) at a wavelength of 550 nm.

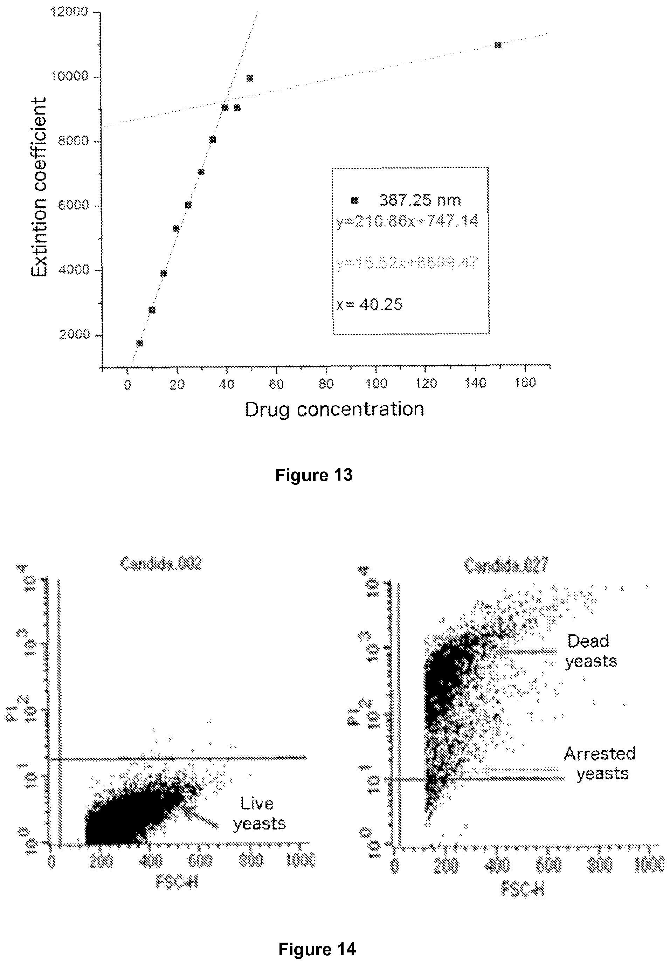

Antifungal activity *in vitro.* The antifungal activity of the polyene macrolide derivate of the present invention was determined by flow cytometry method earlier described (Pinto et al., J. Medical Microbiology, 2009; 58, 1454-1462; Pina-Vaz and Rodriguez, Methods Mol Biol. 2010; 638:281-289). For the test two strains of *Candida albicans* (ATCC 10231 and 752) and *Candida kruzei* were used. A cellular suspension of 1 × 10⁶ UFC/mL was used, which has sown in 96-well plates. The suspension was incubated with macrolide A21 dilutions of 0.01, 0.1, 1, 10 and 100 µM and amphotericin B for 24 h at 37°C under aerobic conditions. As controls were used: untreated yeasts (control of living cells), yeasts exposed to UV radiation (arrest, inhibition of proliferation), and yeasts exposed at temperature of 100°C (dead yeasts). Cells were collected by centrifugation at 10,000 x g for 10 min, they were washed once in phosphate buffer. 50 mL of propidium iodide 0.1 mg / mL were added to the cell suspension. It was incubated during 30 minutes at room temperature and protected from light. Finally, the samples were analyzed by flow cytometry (Becton-Dickinson Facsc Calibur, laser argon 480 nm). The intrinsic parameters and fluorescence in the FL2 channel (yellow/orange fluorescence) for FUN and channel FL3 (red fluorescence, filter 630 nm) for propidium iodide were acquired and registered on a logarithmic scale for a minimum of 7500 events per sample. For data analysis, the quadrants were adjusted in a plot of data lines of fluorescence intensity of samples. In the lower quadrant the live yeast was adjusted (control); on the left upper quadrant the dead yeast, and in the middle the arrested yeasts (inhibition of proliferation). These quadrants were used to quantify the percentile of cells showing altered fluorescence compared to drug-free controls. Figure 14 shows a representative dot plot of the controls used.

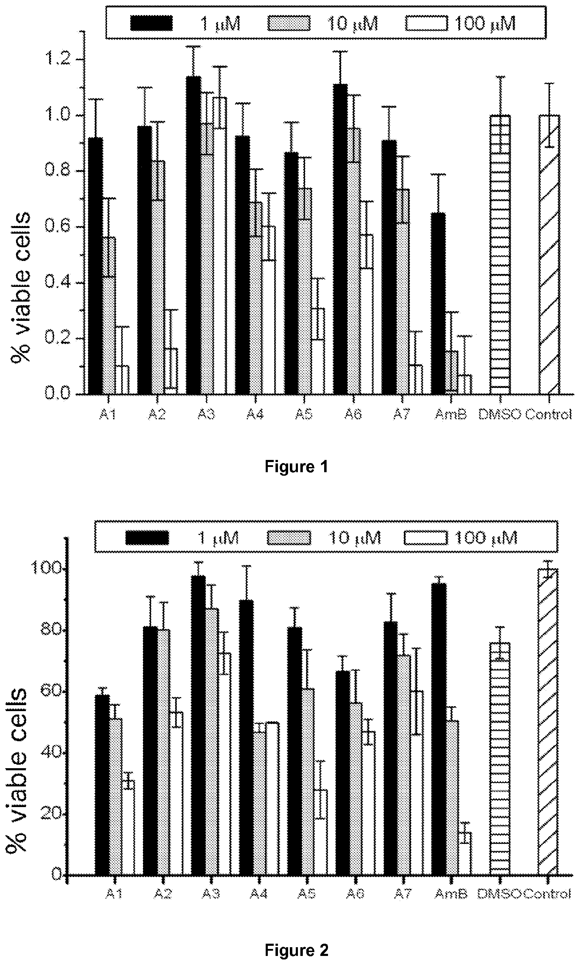

**Example 11. Effect of the compounds of the invention in the viability of *Saccharomyces cerevisae* FY833 cells (SC) at various concentrations.** The effect of various compounds derived from substitution in the COO group of Amphotericin (AmB) on SC cells at different concentrations is presented in figure 1 and compared with the effect of Amphotericin and of dimethyl sulfoxide (DMSO) used as solvent. Clearly, all compounds except the amide 3 (N,N-diisopropylamide) have an inhibitory capacity but with reduced potency compared to the reference, where amide 1 (N-benzylamide) and amide 7 (N-(D)-tryptophanamide) are the most similar. This result is similar to other derivatives of AmB (Chéron et al Biochem. Pharmacol. 1988,827; Carmody M. et al. J. Biol. Chem. 2005,34420).

**Example 12. Effect of the compounds of the invention in the viability of human renal cells 293Q (ATCC CLR-1573) at different concentrations.** The effect of various compounds derived from substitution in the COO group of Amphotericin (AmB) on human renal cells 293Q (ATCC CLR-1573) at different concentrations is presented in figure 2 and compared with the effect of amphotericin and dimethyl sulfoxide (DMSO) used as solvent. Clearly, all compounds have a collateral cytoxicity lower than the reference, where cytoxicity of amide 1 (N-benzylamide) and amide 7 (N-(D)-tryptophanamide) have the lowest cytoxicity while having simultaneously the highest power of the derivatives with the consequent advantages in the selectivity of the drug.

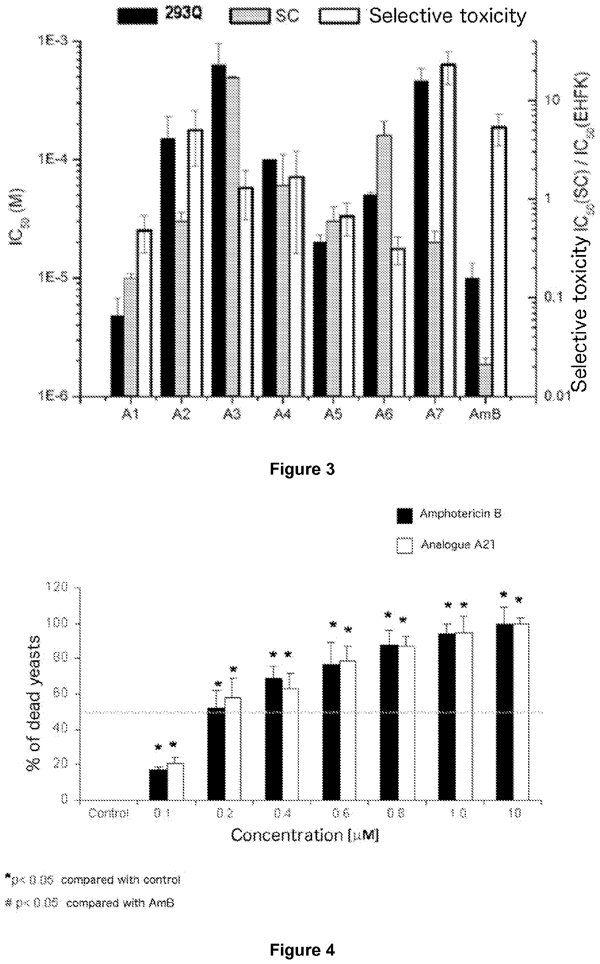

**Example 13. Relative selectivity on the action of the compounds of the invention in the viability of *Saccharomyces cerevisae* FY833(SC) cells compared with the viability in human renal cells 293Q (ATCC CLR-1573) at different concentrations.** The relative effect of various compounds derived from substitution in the COO group of Amphotericin (AmB) on human renal cells (293Q) and fungal cells (SC) at different concentrations is presented in figure 3 and compared with the selectivity of amphotericin. Only the compound denominated amide 7 (N-(D)-tryptophanamide) has a higher selectivity than AmB owed to its much lower action on renal cells. The advantage of selectivity is a factor 4.

**Example 14. Comparison of the action of the compounds of the invention on the lipid bilayer.** Table 4 shows the action of various compounds of the invention in unilamellar lipid bilayers formed with chicken egg lecithin containing 30 mol % of cholesterol at 27°C in an electrolyte solution of 2 M KCl, 1 mM CaCl₂, 10 mM Hepes, and pH 8.0. The activity is determined by the presence of the different channels formed by polyenes in the lipid bilayer (Cotero, *et al., supra*; Venegas, *et al., supra).* It presents the percentile probability that the various opening channels show for each compound. These values were determined by the technique of unit channel delineated above. All compounds show a more reduced activity in the cholesterol membrane than the activity of AmB, and there is some correlation with the activity of compounds in the cells 293Q. Although the compound denominated amide 1 has a low activity of transmembranal channels and a cytoxicity even higher than AmB, indicating that it is another cause and not the formation of channels which produces it. The low cytoxicity of the compound denominated amide 3 is correlated with a more reduced presence of channels in membranes with cholesterol, although its low potency in fungal cells suggests that the same happens in these cells. The low cytoxicity of the compound denominated amide 7 is correlated with a more reduced activity of transmembranal channels and a greater selectivity which is related with a better discrimination of the membranes with cholesterol or ergosterol.

**Table 4. Percentile probability of the opening of various channels in lipid bilayers of lecithin of chicken egg, which are formed by some of the compounds of the invention.**

| **Opening probability** | | | | | | | | |
|---|---|---|---|---|---|---|---|---|
| **[] µM** | 200 | 200 | 200 | 200 | 200 | 200 | 200 | 10 |
| **Type** | A1 | A2 | A3 | A4 | A5 | A6 | A7 | AmB* |
| **I** | 2 % | 5 % | < 1 % | 7 % | 10 % | 23 % | 14 % | 0.59 % |
| **II** | 1 % | < 1 % | N.O. | 3 % | 2 % | 2 % | 7 % | 0.26 % |
| **III** | < 1 % | < 1 % | N.O. | 1 % | < 1 % | < 1 % | < 1 % | 0.08 % |
| **IV** | < 1 % | < 1 % | N.O. | < 1 % | < 1 % | < 1 % | < 1 % | 4.06 % |
| **V** | < 1 % | < 1 % | N.O. | < 1 % | < 1 % | < 1 % | < 1 % | 1.81 % |
| **VI** | < 1 % | < 1 % | N.O. | < 1 % | < 1 % | N.O. | N.O. | 0.93 % |

| | | | | | | | | |
|---|---|---|---|---|---|---|---|---|
| * Venegas, *et al., supra.* N. O. = Not observed | | | | | | | | |

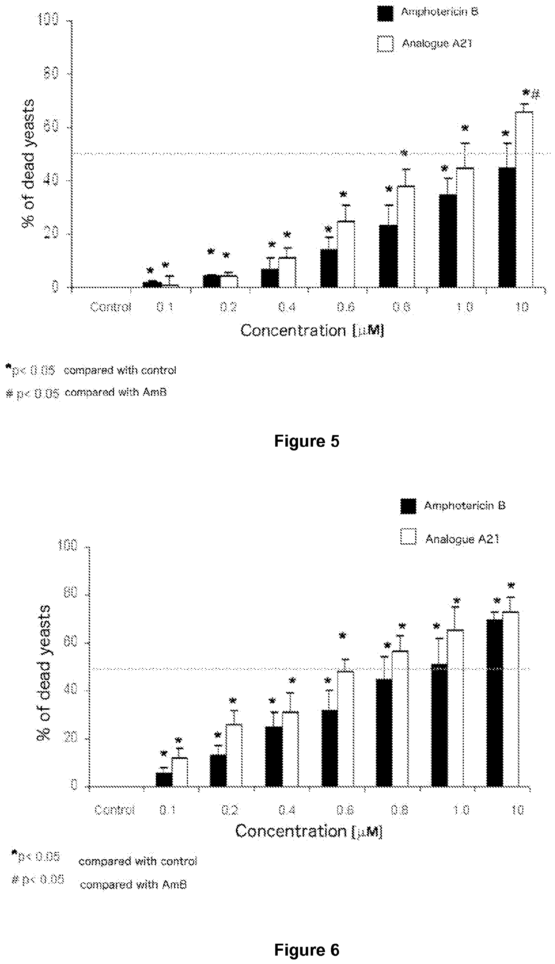

**Example 15. Antifungal activity of the derivative denominated amide A21 compared to AmB for different strains of *Candida albicans.*** Figures 4, 5 and 6 show the antifungal activity of AmB and the compound of the invention denominated A21 as percentage inhibition of populations of different strains of *Candida albicans* with different concentrations of the compounds. In the strain sensible to AmB it is seen that the antifungal activity of amide A21 and of AmB are similar, and in the resistant strains the action of the compound of the invention amide A21 has a considerably greater potency.

**Example 16. Comparison of fungicide concentration 50 (CI50) of some of the compounds of the invention on various strains of *Candida albicans.*** Table 5 shows the fungicide concentration 50 for various derivatives of AmB in strains sensitive and resistant to AmB. Clearly, most of the derivatives have a reduced antifungal potency when compared with AmB, except the amide 10 or derivative A21, which is very similar on the sensible strain and has more potency on the resistant strains.

Hemolytic assay. The hemotoxicity of the polyene macrolide derivative of the present invention was determined by measuring the toxicity to human erythrocytes (values HE50) using a method established by Cybulska, et al. (Cybulska, B., Borowski, E. and Gary-Bobo, M. Relationship between ionophoric and hemolytic activities of perimycin A and vacidin A, two polyene macrolide antifungal antibiotics. Biochem Pharmacol. 1989; 38(11): 1755-1762). For determining the haematoxicity 2 mL blood were obtained and placed in a tube with EDTA. The number of erythrocytes was counted in a Neubauer chamber, and 1 x 10⁷ cells/mL were placed in test tubes. The erythrocytes were resuspended in 450 µL of KCl 150 mM + Tris 3 mM buffer. The erythrocytes were treated with concentrations of 0.01, 0.1, 1, 10 y 100 µM and incubated in a water bath for 1 hour. They were then centrifuged at 1500 rpm for 15 seconds; 150 µL were taken from the supernatant and placed in 96-well plates; the optical density of each group was measured using a plate reader at a wavelength of 550nm. We included one tube with the drug solvent (DMSO 1%) as internal control. We also included one tube only with erythrocytes and 500 µl of buffer KCl 150 mM + Tris 3 mM, which we consider as a negative control group and a tube with 500 µl of red cells and 35 mM NaCl (positive control).

**Table 5. Antifungal concentrations 50 of some of the compounds of the invention on various strains of Candida albicans**

| **Compounds** | ***Candida albicans* ATCC 752** | ***Candida albicans* ATCC 10231** | ***Candida kruzei*** |
|---|---|---|---|
| | CF50 [µM] | CF50 [µM] | CF50 [µM] |
| Amphotericin B | 1 | 0.20 | >10 |
| A-1 | >100 | >100 | >100 |
| A-2 | >100 | >50 | >100 |
| A-3 | >100 | >50 | >100 |
| A-4 | >100 | >50 | >100 |
| A-5 | >100 | >50 | >100 |
| A-6 | >100 | >50 | >100 |
| A-7 | 8.2 | 1.5 | >10 |
| A-8 | >100 | >50 | >100 |
| A-9 | 6.7 | 1.2 | >10 |
| A21 | 0.67 | 0.28 | 7.5 |

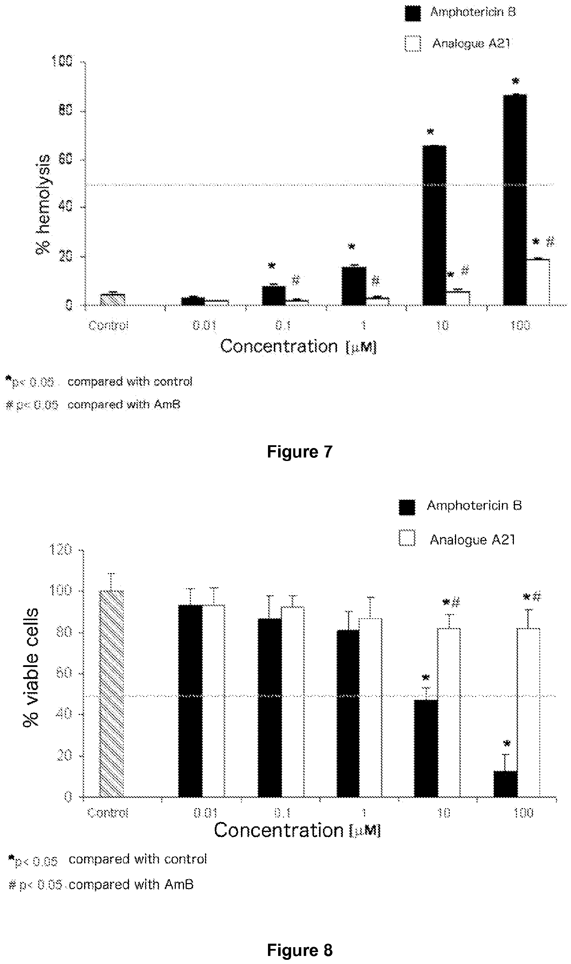

**Example 17. Hemolytic activity of amphotericin and the derivative A21.** Figure 7 shows a comparative hemolytic activity of AmB and the derivative denominated amide A21. Clearly, the hemolytic toxicity of this derivative A21 of the invention is very small compared with that of AmB.

**Example 18. Hemolytic activity of the compounds of the invention.** Table 6 shows the hemolytic activities 50% of the various compounds of the invention. Clearly all of them have a more reduced collateral toxicity, but the compound denominated amide A21 shows a greater reduction.

Essays in kidney cells. For the tests, we used human renal ephythelial cells (293Q) (ATCC Catalog No. CRL-1573). The cells were cultured with Minimum Essential Medium (MEM) (GIBCO BRL) supplemented with 10% fetal bovine serum (FBS) (GIBCO BRL), 2 mM L-glutamine (GIBCO BRL), 1.5 g/L Na₂HCO₃ (Sigma Chemical Co), 0.1 mM nonessential amino acids (Sigma Chemical Co), and 1 mM sodium pyruvate (In vitro, S.A.). For cytoxicity tests, we applied the technique of bromide 3(4,5-dimethylthiazol-2-yl)-2,5-diphenyl tetrazolium (MTT) (Sigma Chemical Co), which is based on the conversion of the dye to an insoluble precipitate called formazan. This test is used as an indicator of mitochondrial function in living cells which measures the metabolic capacity in cells and serves as an indicator of cytotoxicity. (Wang, H.Z., Chang, C.H., Lin, C.P., Tsai, M.C). Using MTT viability assay to test the cytotoxicity of antibiotics and steroid to cultured porcine corneal endothelial cells. J. Ocul. Pharmacol. Ther.1996; 12: 35-43). For the essay 10,000 cells were seeded per well in 96-well plates (Corning Incorporated Costar) in the medium already indicated and incubated for 24 h at 37°C and 5% CO₂. We included different treatment concentrations (0.01, 0.1, 1, 10 and 100 µM) of the polyene macrolide derivative of the present invention and amphotericin B; we also included a group treated with 1% DMSO. After incubation, cells were treated and incubated again for 24 h. The medium was carefully aspirated and replaced with 200 µl fresh medium, adding 50 µl MTT solution for a total volume of 250 µl. They were then incubated for 4 hours in the above conditions. The medium was carefully removed with MTT and 200 µl DMSO and 25 µl Sorense buffer (0.1 M glycine + 0.1 M NaCl at pH 10.5) was added to each well. Then the plate was mixed until the formazan crystals dissolved; it was finally read in a reader plate (Microplate Limaning System Ultramark, Biorad) at a wavelength of 550 nm.

**Table 6. Hemolysis 50 of the various compounds of the invention on human erythrocytes**

| **Compound** | **HE50 [µM]** |
|---|---|
| AmB | 7 |
| A-1 | 52 |
| A-2 | 45 |
| A-3 | 53 |
| A-4 | 46 |
| A-5 | 42 |
| A-6 | 58 |
| A-7 | 80 |
| A-9 | 89 |
| A21 | 409 |

**Example 19. Comparative effect of amphotericin B and the compound of the invention denominated A21 on the viability of human renal cells 293Q (ATCC CLR-1573).** Figure 8 shows the cytotoxic activity on human renal cells produced by amphotericin B and the derivative A21. Clearly, the collateral cytotoxicity of compound A21 is very low compared with AmB. Moreover, it is impossible to obtain the 50% toxicity of the analogue owed to the high concentration that would be necessary.

**Example 20. Cytotoxic activity at 50% of the compounds of the invention on human renal cells.** Table 7 shows the collateral cytotoxic activity of inhibition (DT₅₀) that have the various compounds of the invention on human renal cells 293Q. While all the compounds show a 50% inhibition at higher concentrations than AmB, this reduction of collateral toxicity is much higher.

**Table 7. Collateral cytotoxic activity on human renal cells 293Q at 50% produced by the compounds of the invention**

| **DT50** | |
|---|---|
| **Compound** | **CitE50 [µM]** |
| AmB | 9.7 |
| A-1 | 100 |
| A-2 | 120 |
| A-3 | 180 |
| A-4 | 80 |
| A-5 | 100 |
| A-6 | 120 |
| A-7 | 220 |
| A-9 | 160 |
| A21 | >500 |

Unit channel studies. Unit channel studies were performed according to procedures described earlier (Cotero, *et al*., *supra,* Venegas, *et al., supra).* Required liposomal suspensions were prepared using lecithin with cholesterol or ergosterol. These suspensions were placed in an ultrasonic bath for 15 minutes to produce unilamellar vesicles. Subsequently, the suspension was placed in a 100 µl eppendorf and a lipid bilayer was formed on the tip of a micro-electrode. The unit channels of amphotericin and of the invented compounds were incorporated to this bilayer. The unit channel current of the various compounds was recorded with an electrometer, and then these records were analyzed by determining the activity of the compounds and the characteristics of the channels formed.

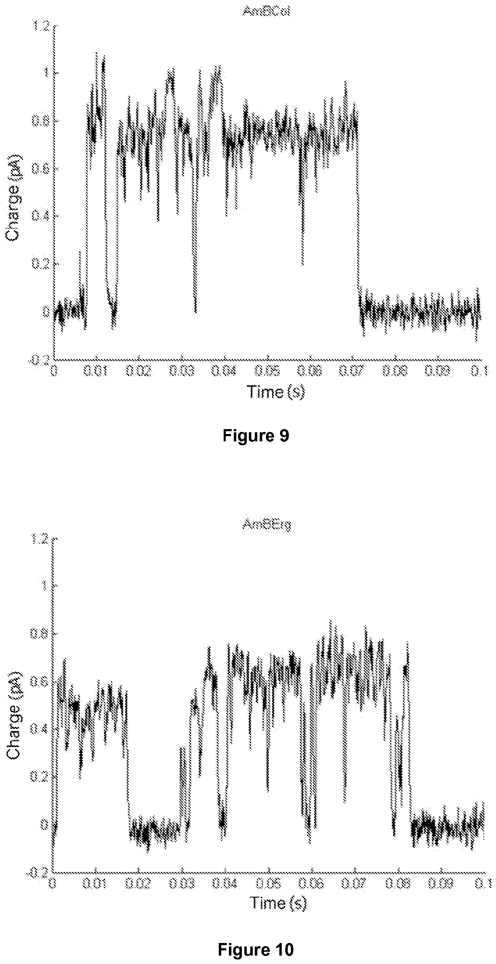

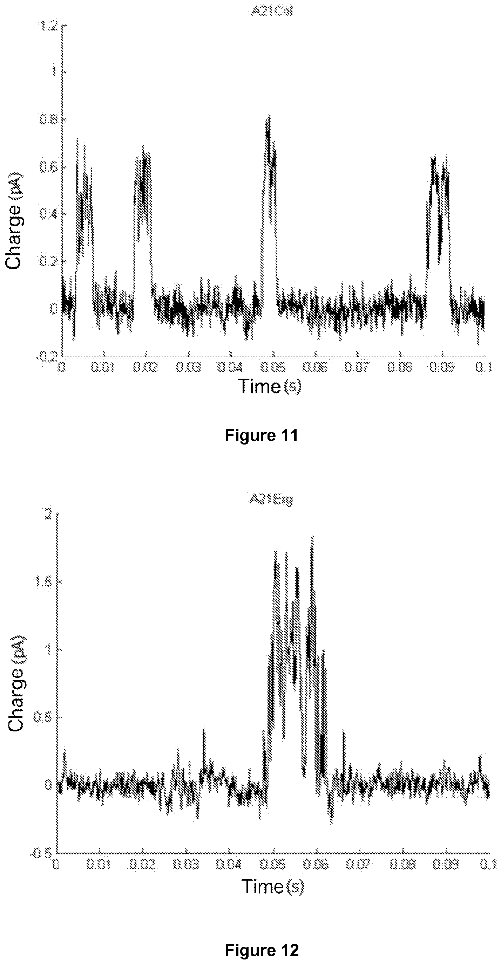

**Example 21. Determination of the transmembranal channels in the lipid bilayer produced by AmB and derivative 21.** Given the remarkably different activity of AmB and derivative 21 on the cells containing cholesterol unlike the similar activity in cells with ergosterol, the activities of both compounds were determined in bilayers of lecithin of chicken egg containing 30% molar cholesterol at 30°C in an electrolyte solution of 2 M KCl, 1 mM CaCl₂, 10 mM Hepes, pH 8.0 at very different concentrations of polyenes. The activity is determined by the presence of the various channels formed by polyenes in the lipid bilayer (Cotero, *et al., supra,* Venegas, *et al., supra).* Figures 9, 10, 11, and 12 show examples of those channels in various membranes. There is different kinetics for the channels, and therefore the activity resulting from the different compounds in different membranes has the behavior observed in pharmacology. To determine this difference we calculated the average conductance that the different membranes acquire in time when incorporating the compound. This conductance is the effect of all the channels present and was determined by the unit channel technique earlier described. Table 8 shows the conductances for the different cases and the relative activity, which correlates with an improved selectivity observed in pharmacology. In these studies derivative A21 showed an increased activity in the membranes with ergosterol than AmB and a not so great activity loss in membranes with cholesterol as showed by pharmacology; the final result in increased selectivity is remarkable and reflected on what is shown by pharmacology.

**Example 22. Comparison of macrolide polyene solubility of the invention with AmB solubility.** We determined the water solubility of derivative A21 according to Lipinski C.A. et al., (Advances in Drug Delivery Reviews, 2001,46, 3-26). For this, aliquots of the compound of the invention dissolved in DMSO were used to produce different concentrations of the polyene in aqueous solution. The absorption spectrum of the molecule was determined for each concentration, and the intensity of the peak at 420 nm was used as a monitor of the dissolved concentration. The concentration was increased until a saturation of the signal. Figure 13 shows the extinction coefficient as a function of the concentration and a bilinear fit of both schemes, so that the point of intersection indicates the solubility of the compound. The solubility obtained this way is ∼ 40 µg/ml, which compares favorably with the solubility of AmB (10 µg/ml) and represents an advantage for the therapeutic application of the drug, without this representing a considerable reduction in its potency.

**Table 8. Average conductances induced in the membranes of chicken egg lecithin produced by AmB and derivative A21**

| **Compound** | **Concentration (µM)** | **Average conductance (fS) cholesterol** | **Average conductance (fS) ergosterol** | **Selectivity** | **Increase in selectivity** |
|---|---|---|---|---|---|
| AmB | 6 | 26.6 ± 9.9 | | 66 | 1 |
| | 2 | | 600 ± 210 | | |
| A21 | 80 | 17.0 ± 22 | | 15718 | 237 |
| | 0.5 | | 1670 ± 630 | | |

The conductance corresponds to all the channels present and therefore, the selectivity is given by the difference in the concentrations applied, and the activity observed.

**Example 23. Comparison of potency and selectivity of recent polyene macrolide derivatives with the compounds of the invention.** Polyene macrolide derivatives have been recently developed with results showing advantages over those used commercially as AmB or Nystatin. As shown in table 9 the compounds developed have a particular advantage in the drug selectivity by yeast cells with a reduced collateral cytotoxicity. This improvement by a factor of 20 showed by the compounds developed by Carreira and Preobrazhenskaya groups is still increased by the compound A21 of the invention where the selectivity increases by a factor of 40.

**Table 9. Comparison of the action of macrolide polyene derivatives of the present invention with the compounds described by Carreira E. and Preobrazhenskaya on fungal cells, human erythrocytes, and human kidney cells, and the improved selectivity fungi / mammal produced by the compounds of the invention.**

| **Carreira E¹** | | | | |
|---|---|---|---|---|
| **Compound** | ***MIC-Candida albicans* DSY294 (µM)** | **MIC₅₀ Hemolysis (µM)** | **Selectivity B/A** | **Increased selectivity Selectivity (AmB) / Selectivity (compound)** |
| AmB | 0.4 | 4 | 10 | 1 |
| Diamine 3 | 0.2 | 10 | 50 | 5 |
| Diamine ester 9 | 0.25 | 50 | 200 | **20** |
| Diamine amide 10 | 1.0 | 30 | 30 | 3 |

| **Preobrazhenskaya M ²** | | | | |
|---|---|---|---|---|
| **Compound** | **MIC₅₀-*Candida albicans* (10231) (µg/ml)** | **MIC₅₀ Hemolysis (µM)** | **Selectivity B/A** | **Increased selectivity Selectivity (AmB) / Selectivity (compound)** |
| AmB | 0.11 | 7.24 | 65.81 | 1 |
| D06 | 0.08 | 100 | 1250 | **19** |

| **Compounds of the present invention** | | | | |
|---|---|---|---|---|
| **Compound** | **MIC₅₀-*Candida albicans* (10231) (µg/ml)** | **MIC₅₀ Hemolysis (µM)** | **Selectivity B/A** | **Increased selectivity Selectivity (AmB) / Selectivity (compound)** |
| AmB | 0.20 | 6.7 | 33.5 | 1 |
| AP21 | 0.28 | 409 | 1460 | **43** |

| **Compound** | **MIC₅₀-*Candida albicans* (10231) (µg/ml)** | **MIC₅₀ Kidney cells (293Q) (µM)** | **Selectivity B/A** | **Increased selectivity Selectivity (AmB) / Selectivity (compound)** |
|---|---|---|---|---|
| AmB | 0.20 | 9.7 | 48.5 | **1** |
| AP21 | 0.28 | > 500 | > 1785 | >**36** |

| | | | | |
|---|---|---|---|---|
| 1. Carreira E., et al., USPat 2009/0186838, July 23, 2009. 2. Preobrazhenskaya M. et al., J. Med Chem., 2009, 52, 189. | | | | |

## Claims

1. Polyene macrolide derivatives selected from the group comprising:
N-(*S*)-*α*-phenylethylamide of Amphotericin B (Formula IV);
N-(*R*)-*α*-phenylethylamide of Amphotericin B (Formula V);
N-(*L*)-tryptophanamide of Amphotericin B (Formula VI);
N-(*D*)-tryptophanamide of Amphotericin B (Formula VII);
N-Histaminamide of Amphotericin B (Formula VIII); 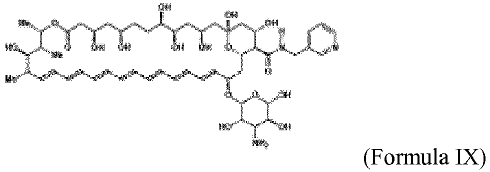 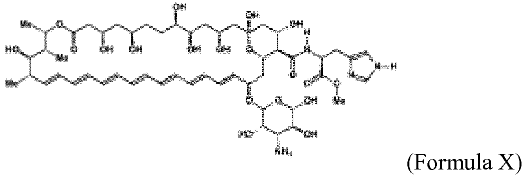 or a pharmaceutically acceptable salt thereof.

2. Polyene macrolide derivatives according to claim 1 wherein said derivative is N-Histaminamide of Amphotericin B (Formula VIII).

3. A pharmaceutical composition with antibiotic properties comprising at least one polyene macrolide derivative or a pharmaceutically acceptable salt thereof according to claim 1 or 2 and a pharmaceutically acceptable carrier.

4. A pharmaceutical composition with antibiotic properties for combination therapy comprising at least one polyene macrolide derivative or a pharmaceutically acceptable salt thereof according to claim 1, an adjuvant agent and a pharmaceutically acceptable carrier.

5. A pharmaceutical composition according to claim 4, wherein the adjuvant agent is selected from the group comprising antifungal, antipyretics, antihistamines and antiemetics compounds.

6. A unit dosage form for pharmaceutical use comprises a polyene macrolide derivative or a pharmaceutically acceptable salt thereof according to claim 1 or a pharmaceutical composition according to claim 5.

7. The pharmaceutical composition according to any of claims 3 to 5 which is formulated for intravenous, subcutaneous, topical, intra peritoneal, inhalation, rectal and / or vaginal administration.

8. A pharmaceutical composition with antibiotic properties comprising at least one polyene macrolide derivative or a pharmaceutically acceptable salt thereof according to claim 2 and a pharmaceutically acceptable carrier.

9. The unit dosage form according to claim 6 which is formulated for intravenous, subcutaneous, topical, intra peritoneal, rectal and / or vaginal administration.

10. A polyene macrolide derivative or a pharmaceutically acceptable salt thereof according to claim 1, for use in a method for inhibiting fungi which comprises contacting a fungus with an effective amount of the polyene macrolide derivative or pharmaceutically acceptable salt thereof under suitable conditions to produce growth inhibition of the fungus.

11. A pharmaceutical composition according to claim 5, for use in a method for inhibiting fungi which comprises contacting a fungus with an effective amount of the polyene macrolide derivative or pharmaceutically acceptable salt thereof under suitable conditions to produce growth inhibition of the fungus.

12. The use of a polyene derivative or a pharmaceutically acceptable salt thereof according to claim 1, or a composition according to claim 5 in the manufacture of a medicament for the treatment and prevention of fungal infections in mammals and the growth inhibition of the fungus.

13. A kit for inhibiting fungi comprising at least one polyene macrolide or a pharmaceutically acceptable salt thereof according to claim 1, or a pharmaceutical composition according to claim 5, and optionally including an adjuvant agent and a pharmaceutically acceptable carrier for inhibiting the growth of fungi.

## Patentansprüche

1. Polyenmakrolidderivate, die aus der Gruppe ausgewählt sind, welche umfasst:
N-(*S*)-*α*-Phenyethylamid von Amphotericin B (Formel IV);
N-(*R*)-*α*-Phenylethylamid von Amphotericin B (Formel V);
N-(*L*)-Tryptophanamid von Amphotericin B (Formel VI);
N-(*D*)-Tryptophanamid von Amphotericin B (Formel VII);
N-Histaminamid von Amphotericin B (Formel VIII); 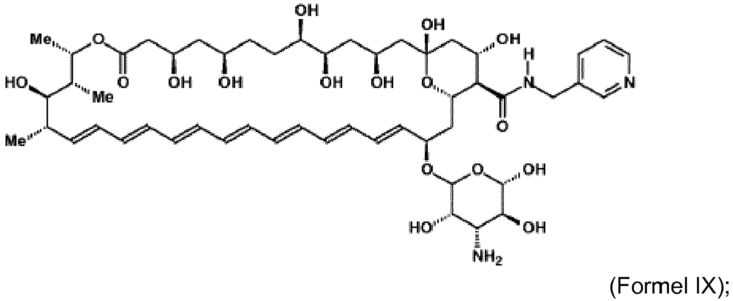 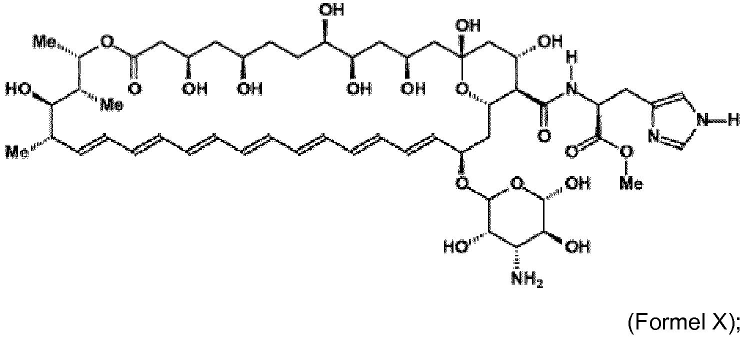 oder ein pharmazeutisch akzeptables Salz davon.

2. Polyenmakrolidderivate nach Anspruch 1, wobei das Derivat ein N-Histaminamid von Amphotericin B (Formel XIII) ist.

3. Pharmazeutische Zusammensetzung mit antibiotischen Eigenschaften, die wenigstens ein Polyenmakrolidderivat oder ein pharmazeutisch akzeptables Salz davon nach Anspruch 1 oder 2 und einen pharmazeutisch akzeptablen Träger umfasst.

4. Pharmazeutische Zusammensetzung mit antibiotischen Eigenschaften für eine Kombinationstherapie, die wenigstens ein Polyenmakrolidderivat oder ein pharmazeutisch akzeptables Salz davon nach Anspruch 1 umfasst sowie ein Hilfsmittel und einen pharmazeutisch akzeptablen Träger.

5. Pharmazeutische Zusammensetzung nach Anspruch 4, wobei das Hilfsmittel aus der Gruppe ausgewählt ist, die antimykotische, antihistaminische und antiemetische Verbindungen umfasst.

6. Einheitsdosisform zur pharmazeutischen Verwendung, die ein Polyenmakrolidderivat oder ein pharmazeutisch akzeptables Salz davon nach Anspruch 1 oder eine pharmazeutische Zusammensetzung nach Anspruch 5 umfasst.

7. Pharmazeutische Zusammensetzung nach einem der Ansprüche 3 bis 5, die zur intravenösen, subkutanen, topischen, intraperitonealen, inhalierenden, rektalen und/oder vaginalen Verabreichung formuliert ist.

8. Pharmazeutische Zusammensetzung mit antibiotischen Eigenschaften, die wenigstens ein Polyenmakrolidderivat oder ein pharmazeutisch akzeptables Salz davon nach Anspruch 2 und einen pharmazeutisch akzeptablen Träger umfasst.

9. Einheitsdosisform nach Anspruch 6, die zur intravenösen, subkutanen, topischen, interperitonealen, rektalen und/oder vaginalen Verabreichung formuliert ist.

10. Polyenmakrolidderivat oder pharmazeutisch akzeptables Salz davon nach Anspuch 1 zur Verwendung bei einem Verfahren zum Hemmen von Pilzen, welches ein Kontaktieren eines Pilzes mit einer wirksamen Menge des Polyenmakrolidderivats oder eines pharmazeutisch akzeptablen Salzes davon unter geeigneten Bedingungen umfasst, um eine Wachstumshemmung des Pilzes bereitzustellen.

11. Pharmazeutische Zusammensetzung nach Anspruch 5 zur Verwendung bei einem Verfahren zum Hemmen von Pilzen, welches ein Kontaktieren eines Pilzes mit einer wirksamen Menge des Polyenmakrolidderivats oder eines pharmazeutisch akzeptablen Salzes davon unter geeigneten Bedingungen umfasst, um eine Wachstumshemmung des Pilzes bereitzustellen.

12. Verwendung eines Polyenderivats oder eines pharmazeutisch akzeptablen Salzes davon nach Anspruch 1 oder eine Zusammensetzung nach Anspruch 5 bei der Herstellung eines Medikaments zur Behandlung und Verhinderung von Pilzinfektionen bei Säugern sowie der Wachstumshemmung des Pilzes.

13. Kit zum Hemmen von Pilzen, das wenigstens ein Polyenmakrolid oder ein pharmazeutisch akzeptables Salz davon nach Anspruch 1 umfasst, oder eine pharmazeutische Zusammensetzung nach Anspruch 5 und optional ein Hilfsmittel enthält sowie einen pharmazeutisch akzeptablen Träger zum Hemmen des Wachstums von Pilzen.

## Revendications

1. Dérivés de macrolides polyènes choisis dans l'ensemble comprenant :
le N-(S)-α-phényléthylamide d'amphotéricine B (formule IV) ;
le N-(R)-α-phényléthylamide d'amphotéricine B (formule V) ;
le N-(L)-tryptophanamide d'amphotéricine B (formule VI) ;
le N-(D)-tryptophanamide d'amphotéricine B (formule VII) ;
le N-histaminamide d'amphotéricine B (formule VIII) ; 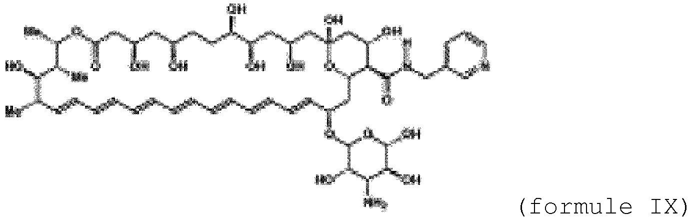 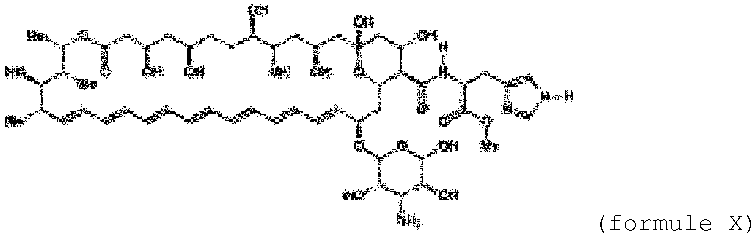 ou un sel pharmaceutiquement acceptable de ceux-ci.

2. Dérivés de macrolides polyènes selon la revendication 1, dans lesquels ledit dérivé est le N-histaminamide d'amphotéricine B (formule VIII).

3. Composition pharmaceutique ayant des propriétés antibiotiques comprenant au moins un dérivé de macrolide polyène ou un sel pharmaceutiquement acceptable de celui-ci selon la revendication 1 ou 2 et un véhicule pharmaceutiquement acceptable.

4. Composition pharmaceutique ayant des propriétés antibiotiques pour une thérapie combinée comprenant au moins un dérivé de macrolide polyène ou un sel pharmaceutiquement acceptable de celui-ci selon la revendication 1, un agent adjuvant et un véhicule pharmaceutiquement acceptable.

5. Composition pharmaceutique selon la revendication 4, dans laquelle l'agent adjuvant est choisi dans le groupe comprenant les composés antifongiques, antipyrétiques, antihistaminiques et antiémétiques.

6. Forme posologique unitaire pour un usage pharmaceutique, comprenant un dérivé de macrolide polyène ou un sel pharmaceutiquement acceptable de celui-ci selon la revendication 1 ou une composition pharmaceutique selon la revendication 5.

7. Composition pharmaceutique selon l'une quelconque des revendications 3 à 5, qui est formulée pour une administration par voie intraveineuse, sous-cutanée, topique, intrapéritonéale, rectale et/ou vaginale, et/ou par inhalation.

8. Composition pharmaceutique ayant des propriétés antibiotiques comprenant au moins un dérivé de macrolide polyène ou un sel pharmaceutiquement acceptable de celui-ci selon la revendication 2 et un véhicule pharmaceutiquement acceptable.

9. Forme posologique unitaire selon la revendication 6, qui est formulée pour une administration par voie intraveineuse, sous-cutanée, topique, intrapéritonéale, rectale et/ou vaginale.

10. Dérivé de macrolide polyène ou un sel pharmaceutiquement acceptable de celui-ci selon la revendication 1, pour une utilisation dans un procédé pour inhiber les champignons, qui comprend la mise en contact d'un champignon avec une quantité efficace du dérivé de macrolide polyène ou de son sel pharmaceutiquement acceptable dans des conditions propices à la production d'une inhibition de la croissance du champignon.

11. Composition pharmaceutique selon la revendication 5, pour une utilisation dans un procédé pour inhiber les champignons, qui comprend la mise en contact d'un champignon avec une quantité efficace du dérivé de macrolide polyène ou de son sel pharmaceutiquement acceptable dans des conditions propices à la production d'une inhibition de la croissance du champignon.

12. Utilisation d'un dérivé de polyène ou d'un sel pharmaceutiquement acceptable de celui-ci selon la revendication 1, ou d'une composition selon la revendication 5, dans la fabrication d'un médicament destiné au traitement et à la prévention d'infections fongiques chez les mammifères et à l'inhibition de la croissance du champignon.

13. Kit pour inhiber des champignons comprenant au moins un macrolide polyène ou un sel pharmaceutiquement acceptable de celui-ci selon la revendication 1, ou une composition selon la revendication 5, et comprenant éventuellement un agent adjuvant et un véhicule pharmaceutiquement acceptable pour inhiber la croissance de champignons.
